# EUROPÄISCHE PATENTSCHRIFT

(11) **EP 3 230 402 B1**
(45) Veröffentlichungstag und Bekanntmachung des Hinweises auf die Patenterteilung: **15.04.2020**
(21) Anmeldenummer: 15797851.1
(22) Anmeldetag: 16.11.2015
(51) Int. Cl.: C09K 11/06, H01L 51/00, H01L 51/50

(54) **ELEKTRONISCHE VORRICHTUNG**
ELECTRONIC DEVICE
DISPOSITIF ÉLECTRONIQUE

(30) Priorität: 09.12.2014 EP 14004144
(43) Veröffentlichungstag der Anmeldung: 18.10.2017
(73) Patentinhaber: Merck Patent GmbH, 64293 Darmstadt (DE)
(72) Erfinder: STOESSEL, Philipp, 60389 Frankfurt am Main (DE); PARHAM, Amir Hossain, 60486 Frankfurt am Main (DE); PFLUMM, Christof, 64291 Darmstadt (DE); JATSCH, Anja, 60489 Frankfurt am Main (DE); KAISER, Joachim, 64289 Darmstadt (DE); BUCHHOLZ, Herwig, 60599 Frankfurt am Main (DE)
(86) Internationale Anmeldenummer: PCT/EP2015/002286
(87) Internationale Veröffentlichungsnummer: WO 2016/091351

(56) Entgegenhaltungen:
- EP-A1- 2 733 762
- EP-A1- 3 229 288
- WO-A1-2014/166572
- WO-A1-2015/135625
- CN-A- 103 985 822

## Beschreibung

Die vorliegende Anmeldung betrifft eine organische Elektrolumineszenzvorrichtung (OLED), enthaltend eine emittierende Schicht, wobei die emittierende Schicht eine Verbindung mit einem geringen Unterschied zwischen den Energien des S₁- und des T₁-Zustands aufweist, sowie weiterhin enthaltend eine aus Lösung aufgebrachte Schicht zwischen emittierender Schicht und Anode, die eine Aminverbindung enthält. Weiterhin betrifft die vorliegende Anmeldung ein Verfahren zur Herstellung einer solchen OLED.

Allgemein wird unter der Bezeichnung OLED eine elektronische Vorrichtung verstanden, welche mindestens ein organisches Material enthält und welche unter Anlegen von elektrischer Spannung Licht emittiert. Der grundsätzliche Aufbau von OLEDs ist dem Fachmann bekannt und unter anderem in US 4539507, US 5151629, EP 0676461 und WO 98/27136 beschrieben.

Die Energien des S₁- und des T₁-Zustands einer Verbindung sind im Rahmen der vorliegenden Anmeldung definiert als diejenigen Energien, die für die betreffenden Zustände der Verbindung durch quantenchemische Rechnungen erhalten werden. Der S₁-Zustand ist dabei der energetisch am niedrigsten liegende angeregte Singulettzustand, und der T₁-Zustand ist der energetisch am niedrigsten liegende Triplettzustand. Wie die quantenchemischen Rechnungen genau durchgeführt werden, ist in den Ausführungsbeispielen beschrieben.

Unter einer Aminverbindung wird im Rahmen der vorliegenden Anmeldung eine Verbindung verstanden, welche mindestens eine Aminogruppe enthält, insbesondere eine Verbindung, welche mindestens eine Arylaminogruppe enthält.

Im Stand der Technik ist bekannt, dass mit bestimmten rein organischen emittierenden Verbindungen, welche nicht phosphoreszieren, sondern fluoreszieren, OLEDs mit sehr guten Effizienzen erhalten werden können. Beispielsweise ist in H. Uoyama et al., Nature 2012, 492, 234, offenbart, dass mit Carbazolyl-Cyanobenzolverbindungen als emittierende Verbindungen OLEDs mit externen Quanteneffizienzen erhalten werden können, die ähnlich gut oder besser als diejenigen sind, die mit phosphoreszierenden Emittern erhalten werden können. Solche emittierenden Verbindungen sind dadurch gekennzeichnet, dass sie einen geringen Unterschied zwischen den Energien des S₁- und des T₁-Zustands aufweisen. Der ihnen zu Grunde liegende Mechanismus der Emission wird als thermisch aktivierte verzögerte Fluoreszenz (thermally activated delayed fluorescence, TADF) bezeichnet. In Mehes et al., Angew. Chem. Int. Ed. 2012, 51, 11311, wird ebenfalls die Verwendung von Verbindungen, welche TADF zeigen, in OLEDs beschrieben.

Die genannten Dokumente betreffen ausschließlich OLEDs, bei denen die organischen Funktionsschichten, insbesondere diejenigen Schichten, welche zwischen Anode und emittierender Schicht angeordnet sind, durch Gasphasenabscheidung aufgetragen wurden. Es ist jedoch von hohem Interesse, die TADF-Emitter-Technologie auch auf OLEDs anzuwenden, welche aus Lösung aufgetragene Schichten aufweisen. Insbesondere ist es dabei von Interesse, dass wenigstens die Schichten zwischen Anode und emittierender Schicht aus Lösung aufgetragen werden. Die CN103985822 offenbart OLEDs in welchen ein Polymer aus Lösung zwischen Anode und emittierender Schicht aufgetragen wird und die emittierende Schicht ein TADF-Material beinhaltet.

Die im Stand der Technik gezeigten Ergebnisse sind jedoch nicht vollständig befriedigend. Es ist insbesondere wünschenswert und verbesserungsbedürftig, dass OLEDs basierend auf dem TADF-Emissionsmechanismus eine hohe Lebensdauer und sehr gute Leistungsdaten aufweisen, insbesondere niedrige Betriebsspannung und hohe Quanteneffizienz. Weiterhin ist es von großer Bedeutung, dass OLEDs basierend auf dem TADF-Emissionsmechanismus nur zu einem geringen Anteil Ausschussprodukt darstellen, d.h. dass nur ein geringer Anteil der hergestellten OLEDs, bevorzugt ein vernachlässigbar kleiner Anteil, nicht funktionsfähig ist. Dies ist insbesondere von Bedeutung, wenn dünne Schichten von weniger als 30 nm Dicke aufgetragen werden.

In diesem Zusammenhang wurde nun überraschend gefunden, dass OLEDs, welche einen TADF-Emitter in der emittierenden Schicht enthalten, und welche eine aus Lösung aufgebrachte Schicht zwischen Anode und emittierender Schicht aufweisen, die eine Aminverbindung enthält, hervorragende Leistungsdaten zeigen. Insbesondere sind die Leistungsdaten verbessert gegenüber ansonsten gleichen OLEDs, welche keine Schicht enthaltend eine Aminoverbindung zwischen Anode und emittierender Schicht aufweisen. Weiterhin wurde überraschend gefunden, dass die oben beschriebenen OLEDs bei der Herstellung einen geringen Anteil Ausschussprodukt aufweisen, d.h. dass nur ein vernachlässigbar kleiner Anteil von ihnen nicht funktionsfähig ist. Hingegen ist bei ansonsten gleichen OLEDs, bei welchen die zwischen Anode und emittierender Schicht angeordnete Schicht nicht aus Lösung aufgebracht ist, der Anteil an Ausschussprodukt deutlich höher.

Gegenstand der vorliegenden Erfindung ist somit eine organische Elektrolumineszenzvorrichtung nach Anspruch 1.

Durch das Aufbringen der Schicht, welche eine Aminverbindung enthält, aus Lösung wird eine anders geformte Oberfläche der Schicht erhalten als wenn die Schicht, welche die Aminverbindung enthält, aus der Gasphase aufgebracht wurde. Insbesondere ist die Oberfläche einer solchen aus Lösung aufgebrachten Schicht planarisiert, d.h sie weist eine glatte, ebenmäßige Oberfläche auf. Wird die Schicht, welche eine Aminverbindung enhält, hingegen aus der Gasphase aufgebracht, so treten die Unebenheiten der Oberfläche der darunter liegenden Schicht in der Oberfläche der neu aufgebrachten Schicht gleichermaßen wieder auf.

Ein weiterer Unterschied liegt in der Ordnung der Moleküle, die in der Schicht vorliegen. Bei einer aus Lösung aufgebrachten Schicht liegen die Moleküle ungeordnet in der Schicht vor. Es tritt nur in äußerst geringem Maße eine gleichmäßige Orientierung der Verbindungen auf. Die Schicht liegt in einem glasartigen Zustand und amorph vor. Hingegen besteht bei einer aus der Gasphase aufgebrachten Schicht eine gewisse Ordnung der Moleküle der Schicht. Der Ordnungsgrad ist insbesondere deutlich höher als bei einer aus Lösung aufgebrachten Schicht. Dies ist in der Literatur beispielweise von X. Xing et al., J. Phys. Chem. C, 2013, 117, 25405-25408, beschrieben.

Bevorzugt ist die emittierende Verbindung eine organische Verbindung. Unter einer organischen Verbindung im Sinne der vorliegenden Erfindung wird eine kohlenstoffhaltige Verbindung verstanden, die keine Metalle enthält. Bevorzugt ist eine organische Verbindung gemäß der vorliegenden Erfindung aus den Elementen C, H, D, B, Si, N, P, O, S, F, Cl, Br und I aufgebaut.

Weiterhin bevorzugt ist die emittierende Verbindung eine lumineszente Verbindung. Unter einer lumineszenten Verbindung im Sinne der vorliegenden Anmeldung wird eine Verbindung verstanden, die in der Lage ist, unter optischer Anregung in einer Umgebung, wie sie in der organischen Elektrolumineszenzvorrichtung vorliegt, bei Raumtemperatur Licht zu emittieren. Dabei weist die emittierende Verbindung bevorzugt eine Lumineszenzquanteneffizienz von mindestens 40 % auf, besonders bevorzugt von mindestens 50 %, ganz besonders bevorzugt von mindestens 60 % und insbesondere bevorzugt von mindestens 70 %. Dabei wird die Lumineszenzquanteneffizienz bestimmt in einer Schicht, wie sie in der organischen Elektrolumineszenzvorrichtung verwendet werden soll. Wie die Bestimmung der Lumineszenzquantenausbeute im Sinne der vorliegenden Erfindung durchgeführt wird, ist im Beispielteil beschrieben (Abschnitt Photolumineszenz-Quanteneffizienz).

Weiterhin ist es bevorzugt, wenn die emittierende Verbindung eine kurze Abklingzeit aufweist. Dabei ist die Abklingzeit bevorzugt ≤ 50 µs. Wie die Bestimmung der Abklingzeit im Sinne der vorliegenden Erfindung durchgeführt wird, ist im Beispielteil beschrieben.

Die betragsmäßige Differenz zwischen den Energien des S₁- und des T₁-Zustands der emittierenden Verbindung beträgt erfindungsgemäß höchstens 0.15 eV. Bevorzugt ist die betragsmäßige Differenz ≤ 0.10 eV, besonders bevorzugt ≤ 0.08 eV, ganz besonders bevorzugt ≤ 0.05 eV.

Die emittierende Verbindung ist eine aromatische Verbindung, die sowohl mindestens einen Donor- als auch mindestens einen Akzeptorsubstituenten aufweist, wobei das LUMO und das HOMO der Verbindung räumlich nur geringfügig überlappen. Was unter Donor- bzw. Akzeptorsubstituenten verstanden wird, ist dem Fachmann prinzipiell bekannt. Geeignete Donorsubstituenten sind insbesondere Diaryl- oder Diheteroarylaminogruppen sowie Carbazolgruppen oder Carbazolderivate, die jeweils bevorzugt über N an eine aromatische Verbindung gebunden sind. Dabei können diese Gruppen auch weiter substituiert sein. Geeignete Akzeptorsubstituenten sind insbesondere Cyanogruppen und elektronenarme Heteroarylgruppen, die auch weiter substituiert sein können.

Beispiele für geeignete emittierende Verbindungen im Sinne der vorliegenden Anmeldung sind die im Folgenden aufgeführten Strukturen.

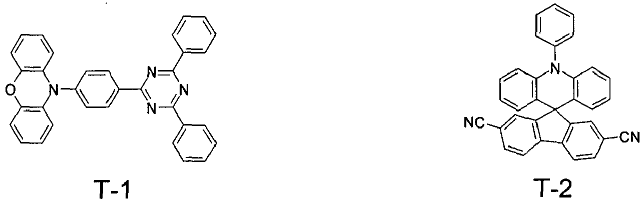

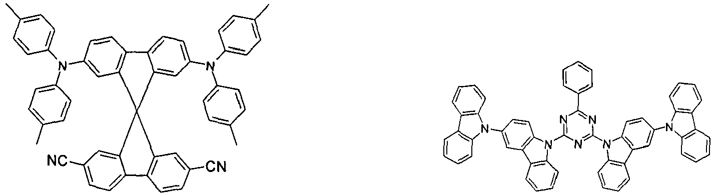

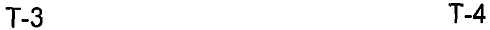

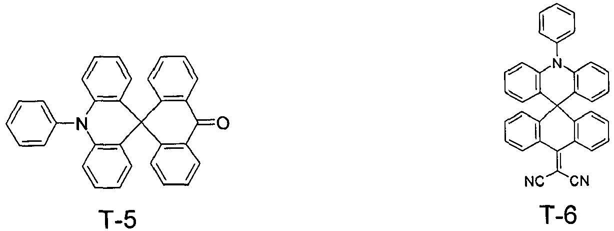

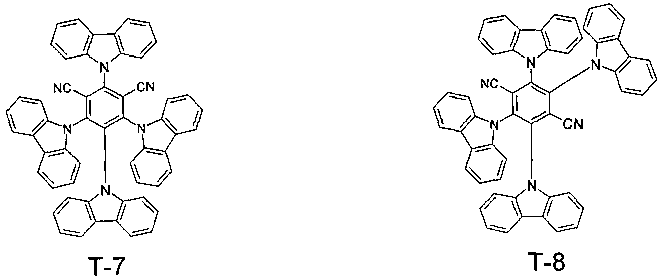

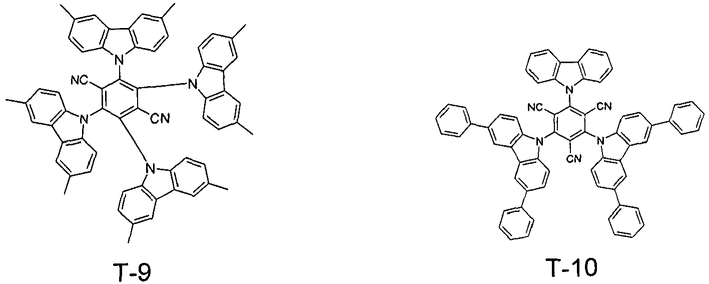

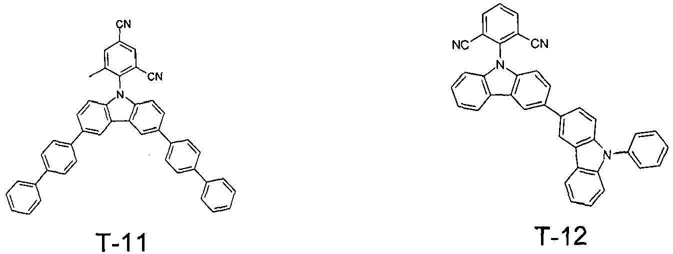

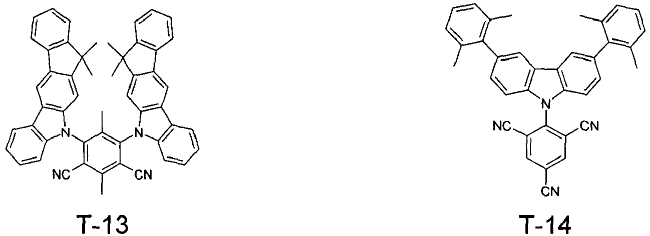

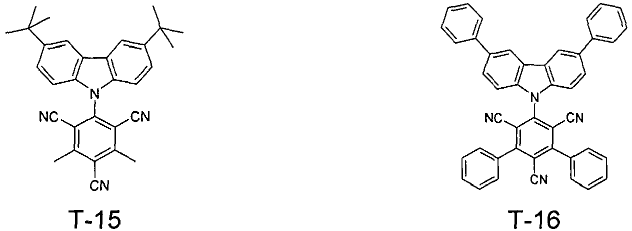

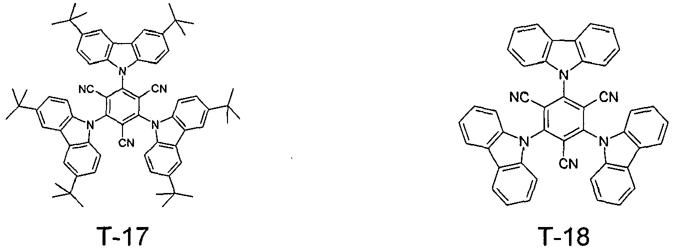

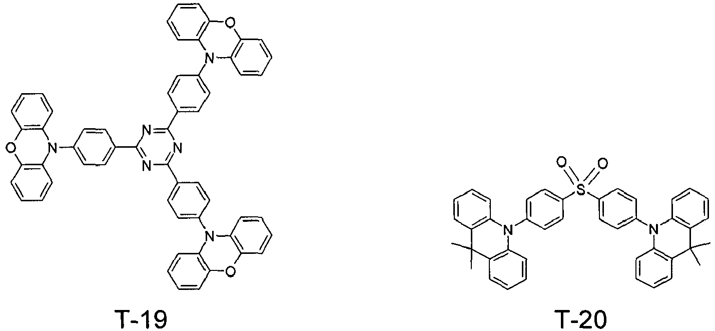

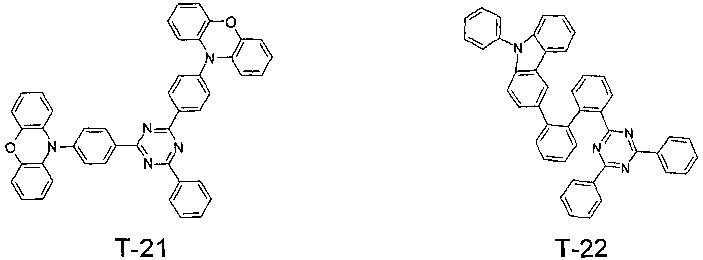

Weiterhin sind im Stand der Technik eine Vielzahl von emittierenden Verbindungen bekannt, die die oben genannte Bedingung für den Abstand zwischen S₁- und T₁-Energieniveau erfüllen, und aus denen der Fachmann auswählen kann, um geeignete emittierende Verbindungen für die in der vorliegenden Anmeldung beschriebene OLED zu erhalten, z.B.: Tanaka et al., Chemistry of Materials 25(18), 3766 (2013), Zhang et al., Nature Photonics Volume 8,p. 326-332, Serevicius et al., Physical Chemistry Chemical Physics 15(38), 15850 (2013), Youn Lee et al., Applied Physics Letters 101(9), 093306 (2012), Nasu et al., ChemComm, 49, 10385 (2013), WO 2013/154064, WO 2013/161437, WO 2013/081088 und WO 2013/011954.

Die emittierende Schicht umfasst bevorzugt neben der emittierenden Verbindung ein oder mehrere Matrixverbindungen, besonders bevorzugt genau ein oder zwei Matrixverbindungen, ganz besonders bevorzugt genau eine Matrixverbindung. Bevorzugt tragen die ein oder mehreren Matrixverbindungen im Betrieb nicht zur Emission der Vorrichtung bei.

Bevorzugt liegt die emittierende Verbindung in der emittierenden Schicht in einem Anteil von 1 bis 25 Vol.-%, besonders bevorzugt von 2 bis 20 Vol.- %, ganz besonders bevorzugt von 4 bis 15 Vol.-%, und am stärksten bevorzugt von 5 bis 12 Vol-% vor. Bevorzugt liegen in diesem Fall außer der emittierenden Verbindung als weitere Verbindungen nur eine oder mehrere Matrixverbindungen in der emittierenden Schicht vor, so dass diese den restlichen Anteil ausmachen.

Es ist bevorzugt, wenn für LUMO(E), also das LUMO-Energieniveau der emittierenden Verbindung, und HOMO(Matrix), also das HOMO-Energieniveau der Matrixverbindung, gilt:
LUMO(E) - HOMO(Matrix) > S₁(E) - 0.4 eV;
besonders bevorzugt:
LUMO(E) - HOMO(Matrix) > S₁(E) - 0.3 eV;
und ganz besonders bevorzugt:
LUMO(E) - HOMO(Matrix) > S₁(E) - 0.2 eV.
Dabei ist S₁(E) die Energie des ersten angeregten Singulettzustands der emittierenden Verbindung.

Es ist weiterhin bevorzugt, dass die Energie des T₁-Zustands der Matrixverbindung der emittierenden Schicht, im Folgenden bezeichnet als T₁(Matrix), höchstens 0.1 eV niedriger ist als die Energie des T₁-Zustands der emittierenden Verbindung, im Folgenden bezeichnet als T₁(E). Besonders bevorzugt ist T₁(Matrix) ≥ T₁(E). Ganz besonders bevorzugt gilt: T₁ (Matrix) - T₁(E) ≥ 0.1 eV, am stärksten bevorzugt T₁ (Matrix) - T₁(E) ≥ 0.2 eV.

Beispiele für geeignete Matrixverbindungen in der emittierenden Schicht sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixverbindungen, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, oder verbrückte Carbazolderivate, z. B. gemäß US 2009/0136779, WO 2010/050778, WO 2011/042107 oder WO 2011/088877.

Bevorzugt zur Verwendung als Matrixverbindungen in der emittierenden Schicht sind elektronentransportierende organische Verbindungen. Besonders bevorzugt sind elektronentransportierende organische Verbindungen, die ein LUMO-Energieniveau von höchstens -2.50 eV aufweisen, besonders bevorzugt höchstens -2.60 eV, ganz besonders bevorzugt höchstens -2.65 eV, und am stärksten bevorzugt höchstens -2.70 eV.

Besonders bevorzugte Matrixverbindungen in der emittierenden Schicht sind ausgewählt aus den Stoffklassen der Triazine, der Pyrimidine, der Lactame, der Metallkomplexe, insbesondere der Be-, Zn- bzw. Al-Komplexe, der aromatischen Ketone, der aromatischen Phosphinoxide, der Azaphosphole, der Azaborole, welche mit mindestens einem elektronenleitenden Substituenten substituiert sind, der Chinoxaline, der Chinoline und der Isochinoline.

Bevorzugt ist die Matrixverbindung der emittierenden Schicht nicht die folgende Verbindung: 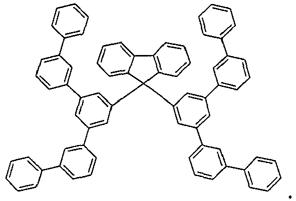 Besonders bevorzugt stellt die Matrixverbindung der emittierenden Schicht keine Wide-Bandgap-Verbindung dar, worunter Verbindungen mit einer Differenz zwischen HOMO-Energie und LUMO-Energie von mindestens 3.5 eV verstanden werden. HOMO und LUMO-Energien werden dabei bestimmt wie in den Ausführungsbeispielen angegeben.

Weiterhin ist die Matrixverbindung der emittierenden Schicht bevorzugt nicht die folgende Verbindung: 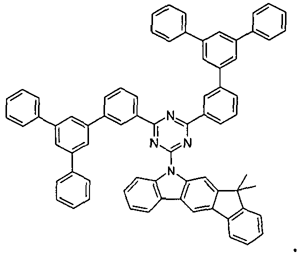 Besonders bevorzugt ist die Matrixverbindung der emittierenden Schicht keine Indenocarbazol-Verbindung.

Die zwischen Anode und emittierender Schicht angeordnete Schicht, welche aus Lösung aufgebracht ist und welche eine Aminverbindung enthält, enthält bevorzugt keine weiteren Verbindungen. Wenn weitere Verbindungen enthalten sind, so sind diese bevorzugt gewählt aus weiteren Aminverbindungen und aus p-Dotanden.

Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, 12, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃. Bevorzugt sind weiterhin Bismut-Komplexe mit elektronenarmen Carboxylat-Liganden, bevorzugt fluorierten Carboxylat-Liganden.

Die zwischen Anode und emittierender Schicht angeordnete Schicht, welche aus Lösung aufgebracht ist und welche eine Aminverbindung enthält, hat eineDicke von mehr als 30 nm. Hierdurch wird eine höhere Zuverlässigkeit der OLEDs erzielt, insbesondere die Ausfallhäufigkeit reduziert.

Bevorzugt sind darunter Triarylaminverbindungen. Unter Triarylaminverbindungen werden Verbindungen verstanden, in denen drei Aryl- oder Heteroarylgruppen an ein Stickstoffatom gebunden sind. Bevorzugt sind dabei Arylgruppen. Ganz besonders bevorzugte Aminverbindungen sind Mono-Triarylaminverbindungen. Darunter werden Verbindungen verstanden, welche nicht mehr als eine chemische Gruppe umfassen, die ein Triarylamin, wie oben definiert, darstellt.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Stand der Technik verwendete niedermolekulare Aminverbindungen sind gewählt aus den Formeln (M-1) bis (M-6) 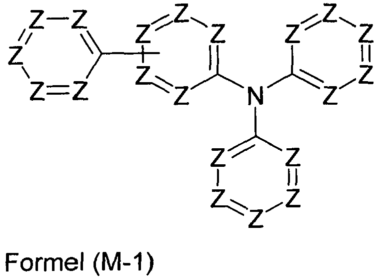 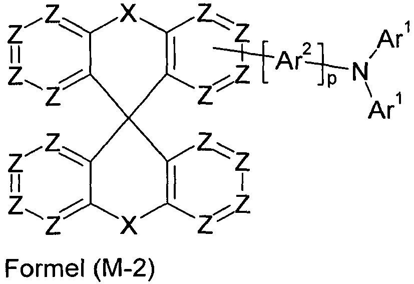 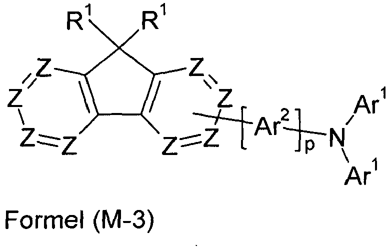 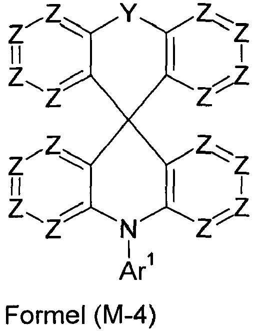 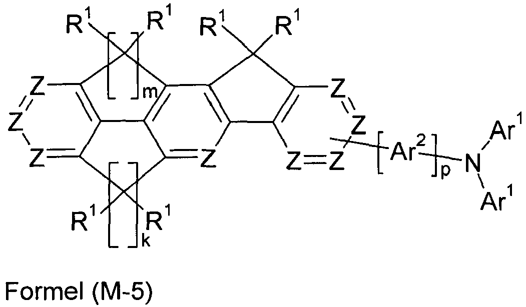 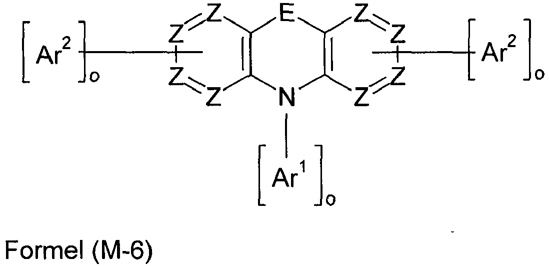 wobei gilt:
- Z: ist bei jedem Auftreten gleich oder verschieden N oder CR¹, wobei Z gleich C ist, wenn ein Substituent gebunden ist;
- X: ist bei jedem Auftreten gleich oder verschieden eine Einfachbindung, O, S, BR¹, C(R¹)₂, Si(R¹)₂, NR¹, PR¹, C(R¹)₂-C(R¹)₂, oder CR¹=CR¹;
- Y: ist eine Einfachbindung, O, S, BR¹, C(R¹)₂, Si(R¹)₂, NR¹, PR¹, C(R¹)₂-C(R¹)₂, oder CR¹=CR¹;
- E: ist O, S, BR¹, C(R¹)₂, Si(R¹)₂, NR¹, PR¹, C(R¹)₂-C(R¹)2, oder CR¹=CR¹;
- Ar¹: ist bei jedem Auftreten gleich oder verschieden ein aromatisches oder heteroaromatisches Ringsystem mit 5 bis 30 aromatischen Ringatomen, das mit einem oder mehreren Resten R¹ substituiert sein kann;
- Ar²: ist ein aromatisches oder heteroaromatisches Ringsystem mit 6 bis 18 aromatischen Ringatomen, das mit einem oder mehreren Resten R¹ substituiert sein kann;
- R¹: ist bei jedem Auftreten gleich oder verschieden gewählt aus H, D, F, C(=O)R^{2,} CN, Si(R²)₃, N(R²)2, P(=O)(R²)2, OR², S(=O)R², S(=O)₂R², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R² substituiert sein können; und wobei eine oder mehrere CH₂-Gruppen in den genannten Alkyl-, Alkoxy-, Alkenyl- und Alkinylgruppen durch -R²C=CR²-, -C≡C-, Si(R²)₂, C=O, C=NR², -C(=O)O-, -C(=O)NR²-, NR², P(=O)(R²), -O-, -S-, SO oder SO₂ ersetzt sein können;
- R²: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R² miteinander verknüpft sein können und einen Ring bilden können; und wobei die genannten Alkylgruppen, aromatischen Ringsysteme und heteroaromatischen Ringsysteme mit F oder CN substituiert sein können;
- o: ist bei jedem Auftreten gleich oder verschieden 0 oder 1, wobei die Summe aller Indices o mindestens gleich 1 ist;
- p: ist gleich 0 oder 1;
- k, m: sind gleich oder verschieden 0 oder 1, wobei die Summe aller Indices k und m gleich 1 oder 2 ist.

Für die oben genannten Formeln (M-1) bis (M-6) gilt bevorzugt, dass nicht mehr als drei Gruppen Z in einem Ring gleich N sind. Es ist allgemein bevorzugt, dass Z gleich CR¹ ist.

Die Gruppe X ist bei jedem Auftreten bevorzugt gleich oder verschieden gewählt aus einer Einfachbindung, C(R¹)₂, O und S, besonders bevorzugt ist sie eine Einfachbindung.

Die Gruppe Y ist bevorzugt gewählt aus O und C(R¹)₂, besonders bevorzugt ist sie O.

Die Gruppe E ist bevorzugt gewählt aus C(R¹)₂, O und S, besonders bevorzugt ist sie C(R¹)₂.

Die Gruppe Ar¹ ist in den oben genannten Formeln bevorzugt bei jedem Auftreten gleich oder verschieden gewählt aus aromatischen oder heteroaromatischen Ringsystemen mit 6 bis 30 aromatischen Ringatomen, die mit einem oder mehreren Resten R¹ substituiert sein können. Besonders bevorzugt ist Ar¹ in den oben genannten Formeln bei jedem Auftreten gleich oder verschieden gewählt aus Aryl- oder Heteroarylgruppen mit 6 bis 18 aromatischen Ringatomen, die mit einem oder mehreren Resten R¹ substituiert sein können.

Beispiele aus dem Stand der Technik sind die folgenden niedermolekularen Aminverbindungen:

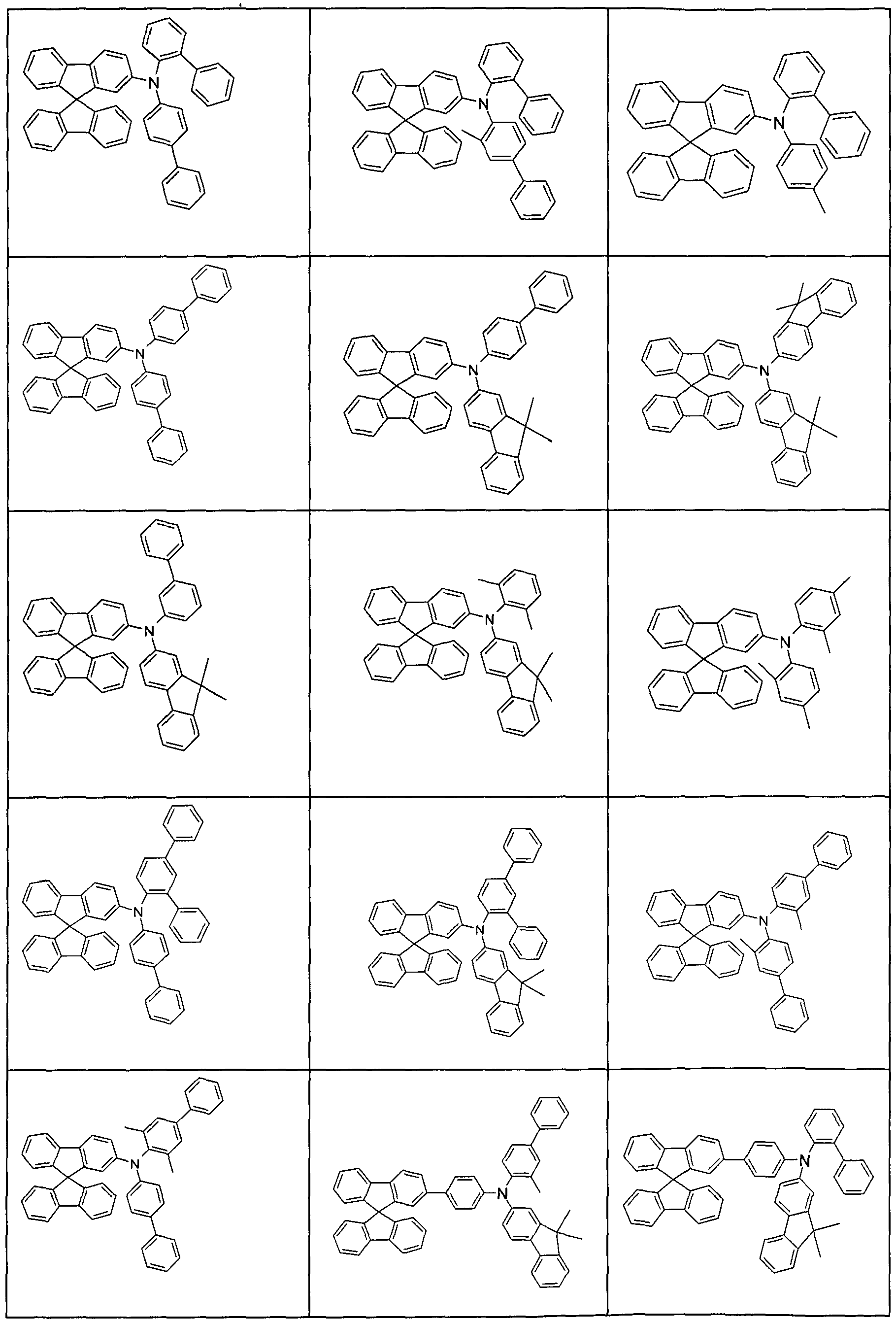

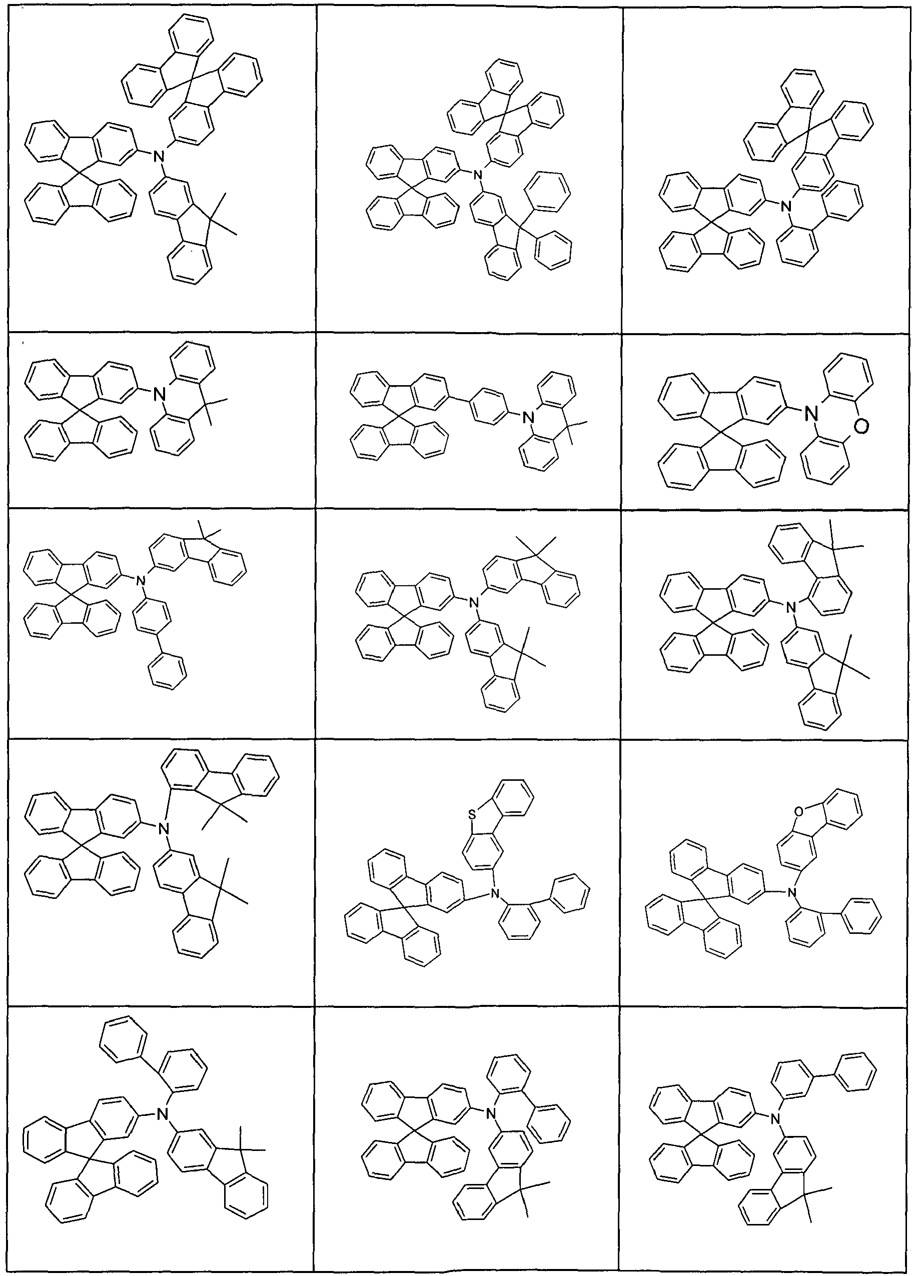

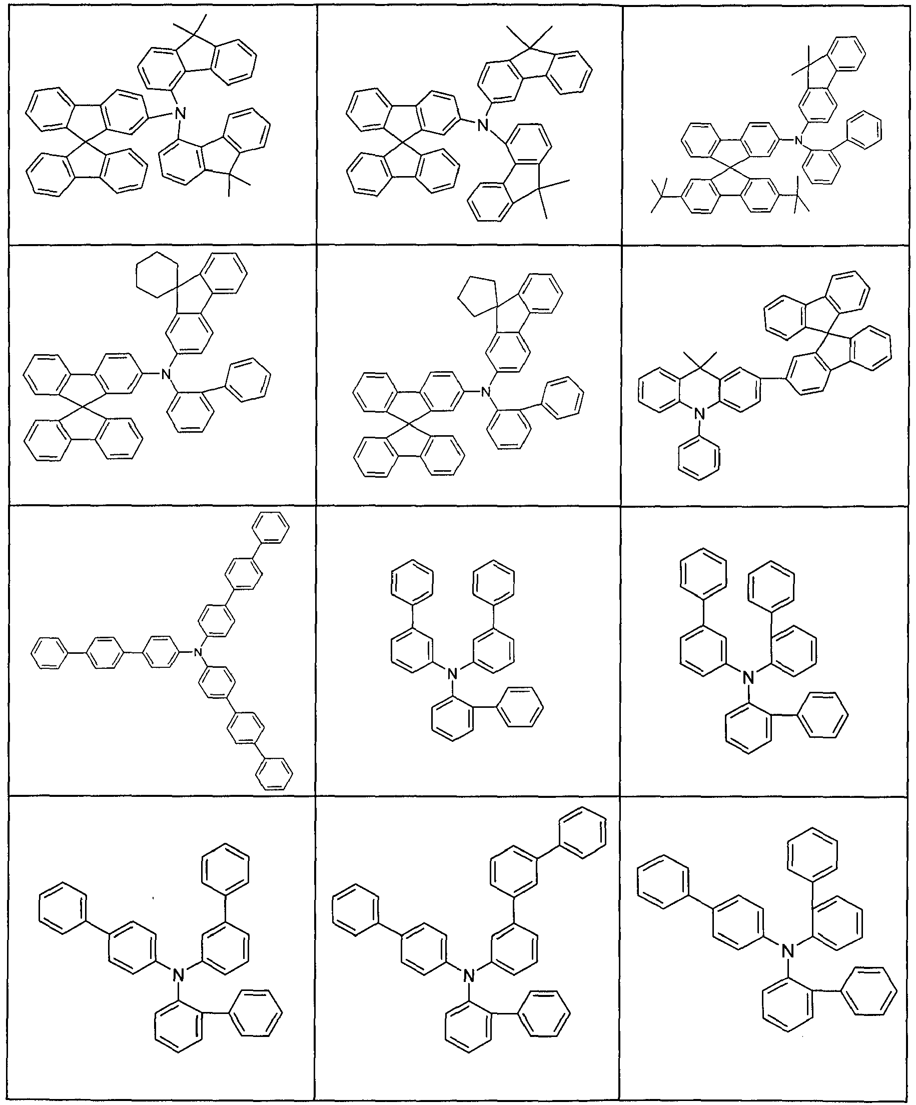

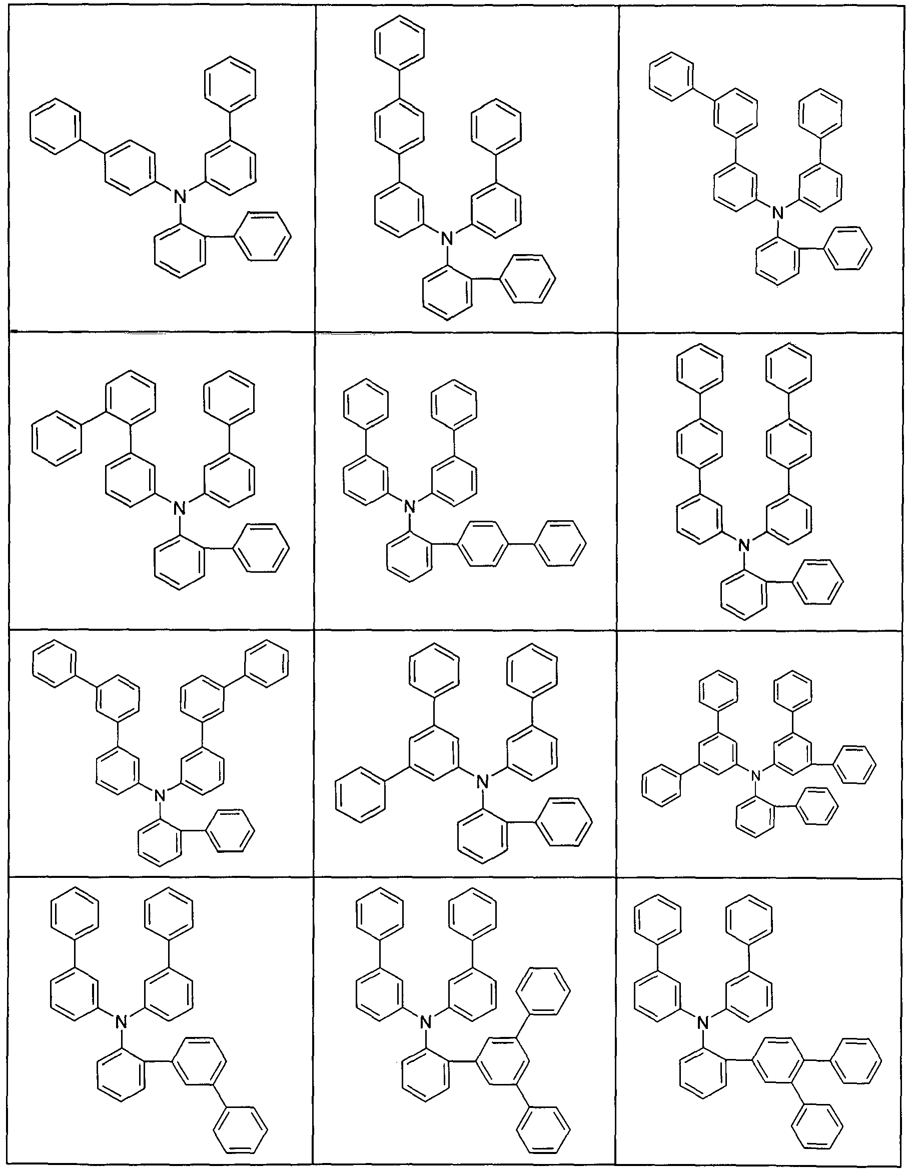

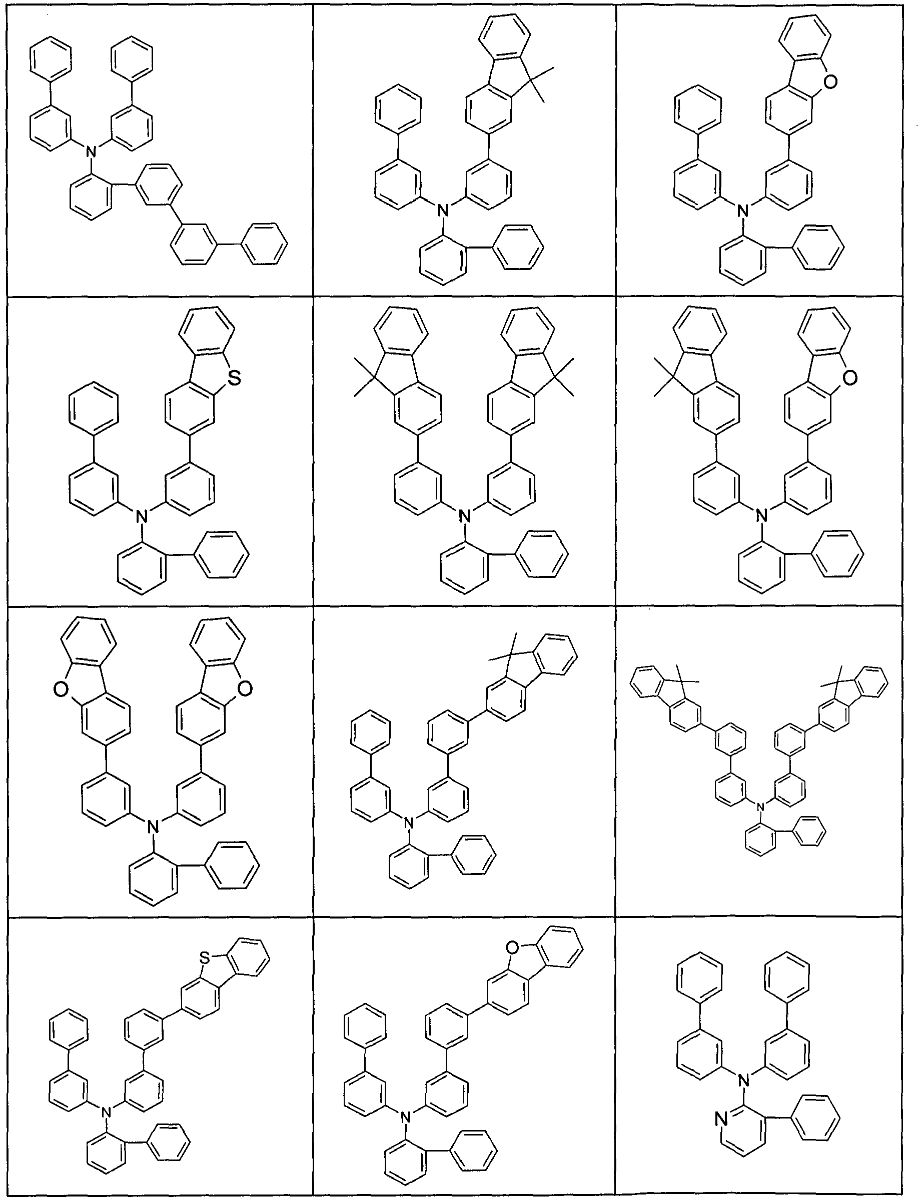

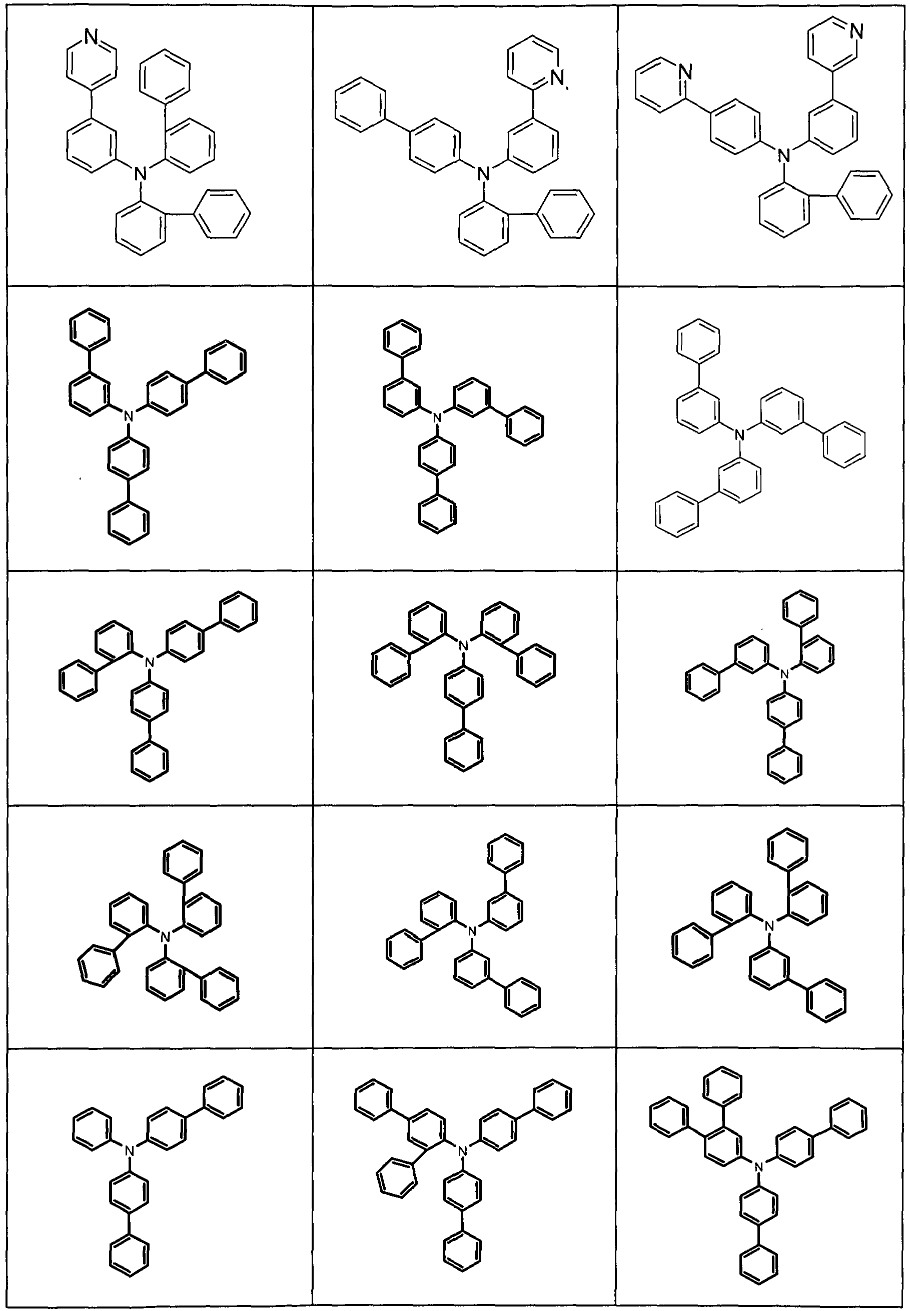

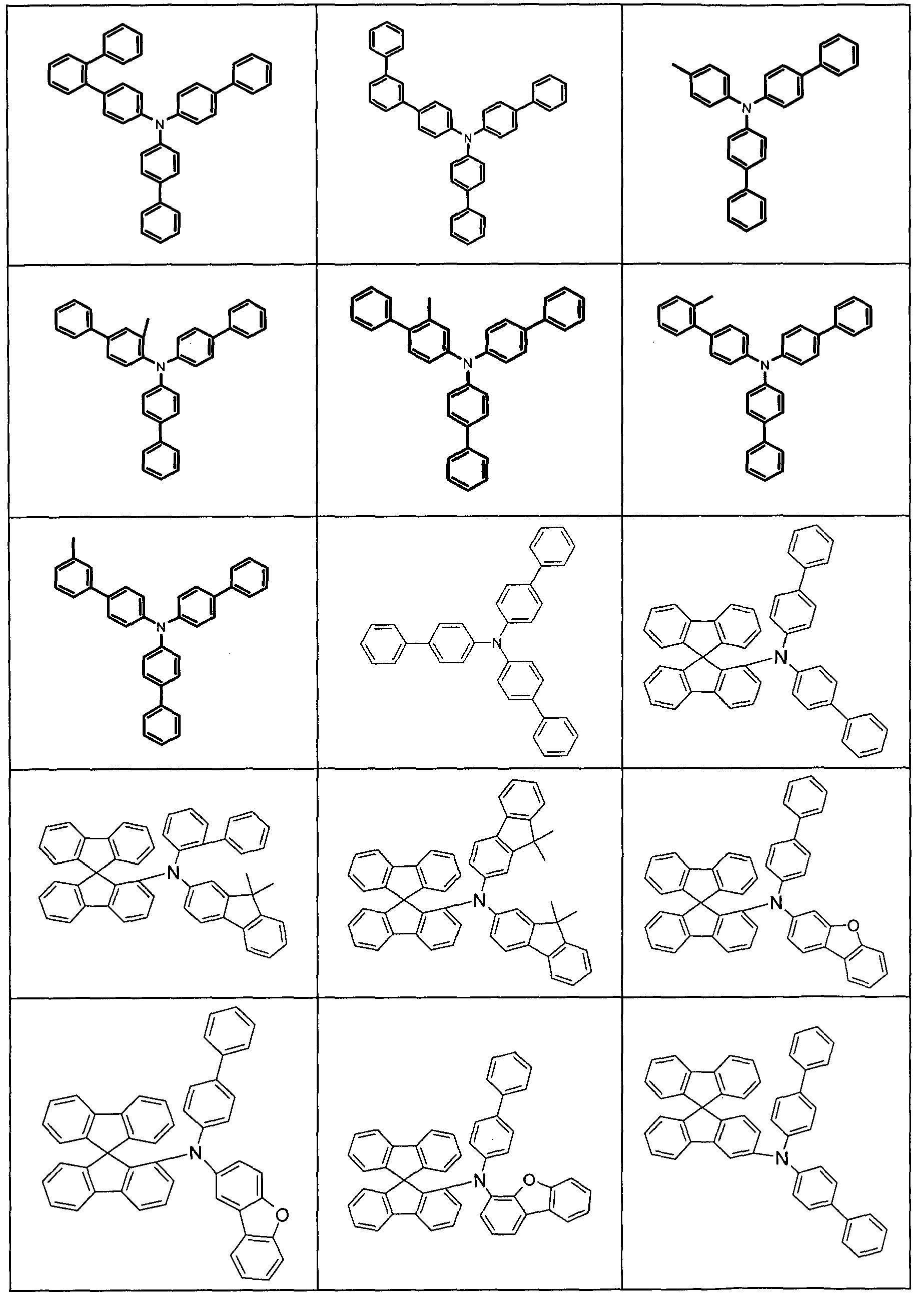

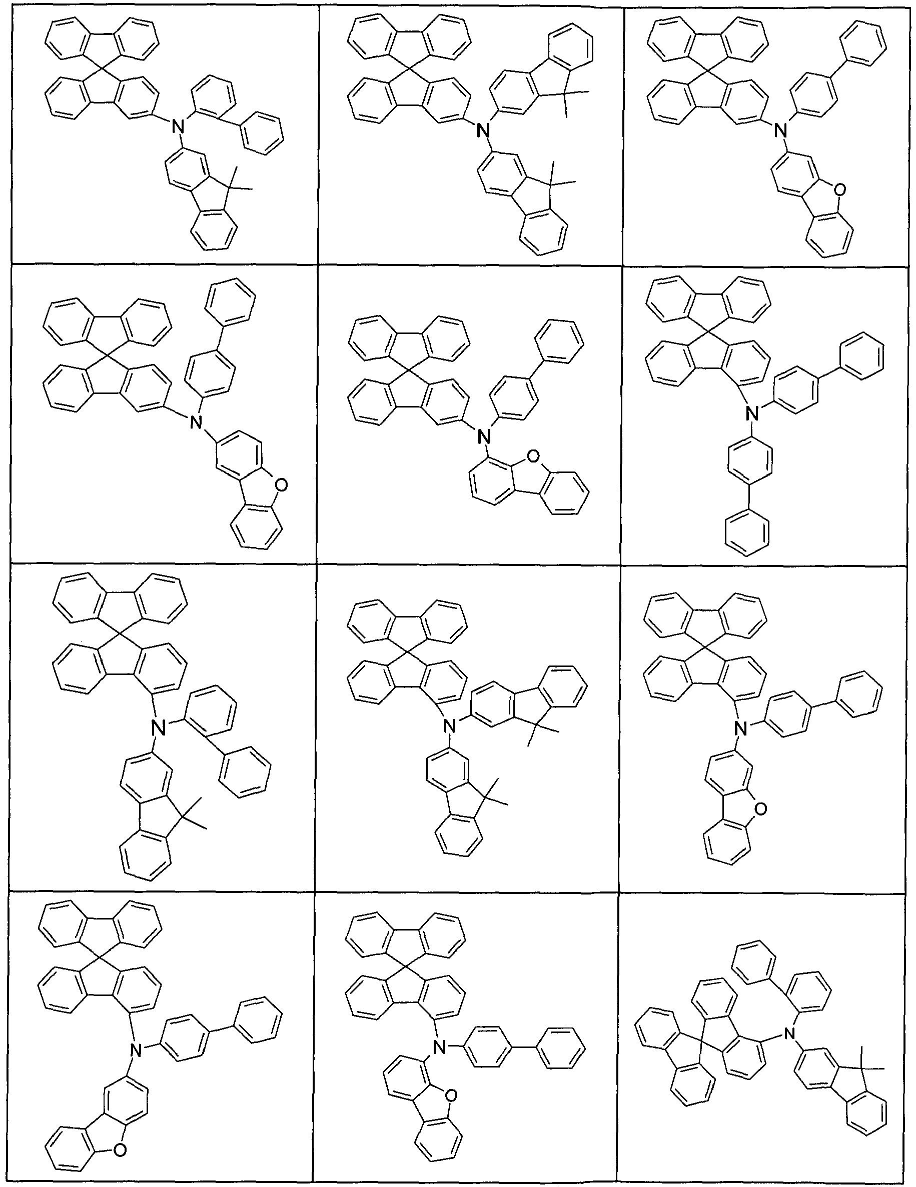

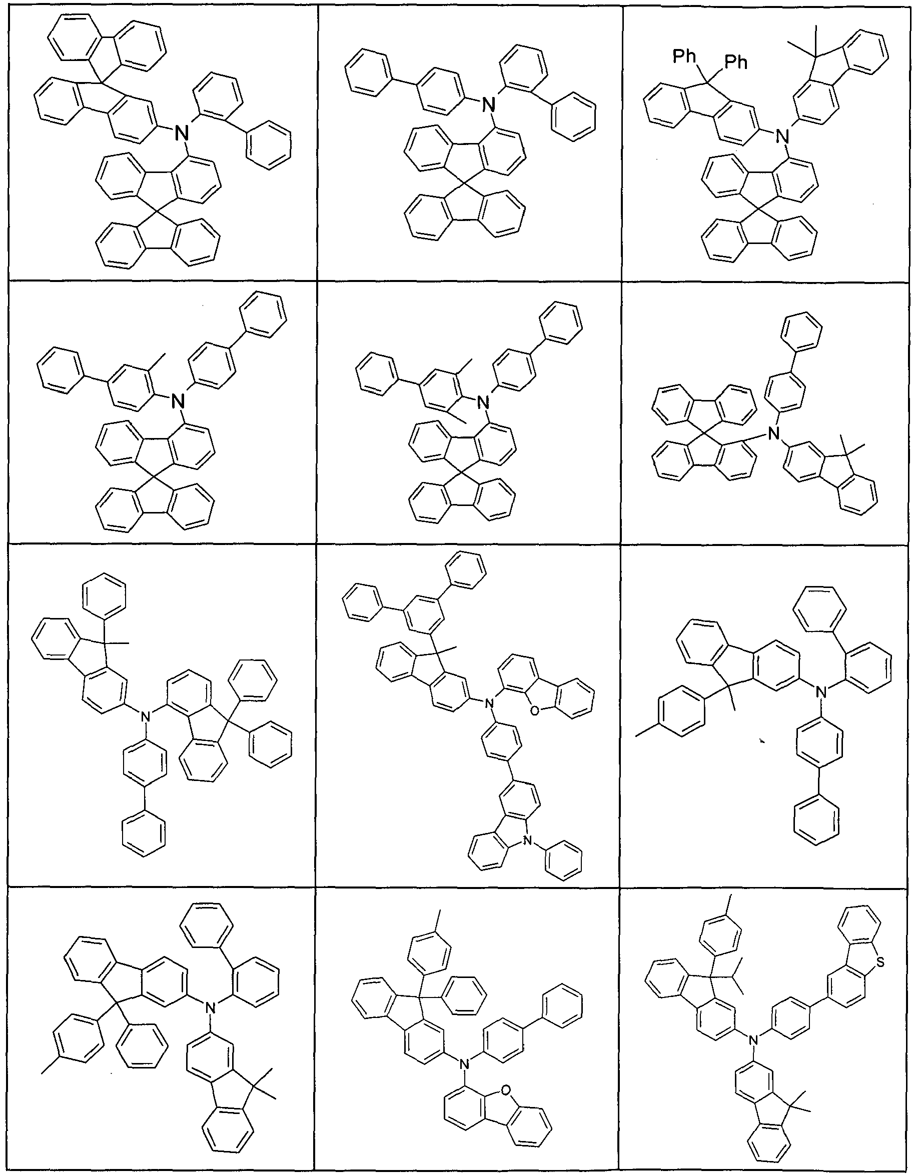

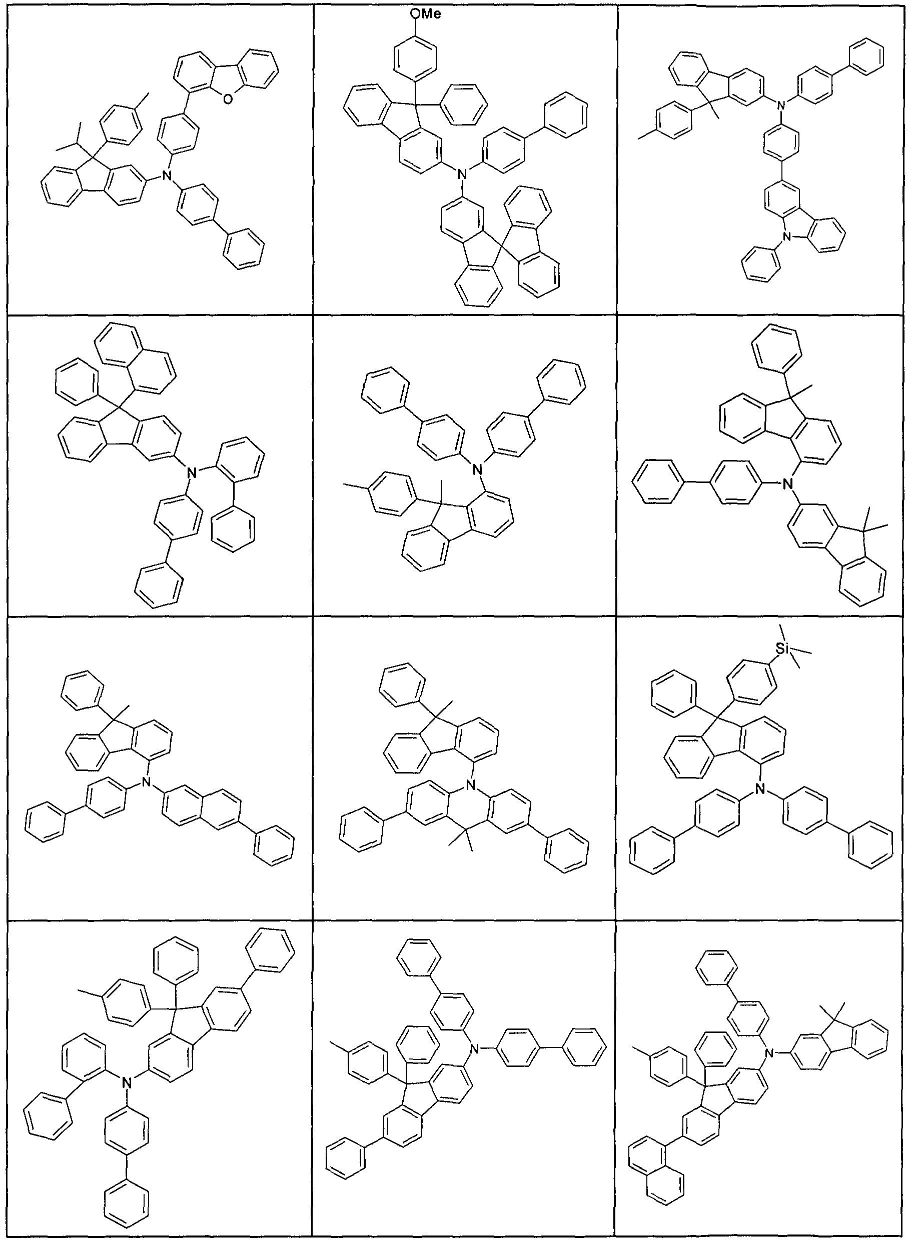

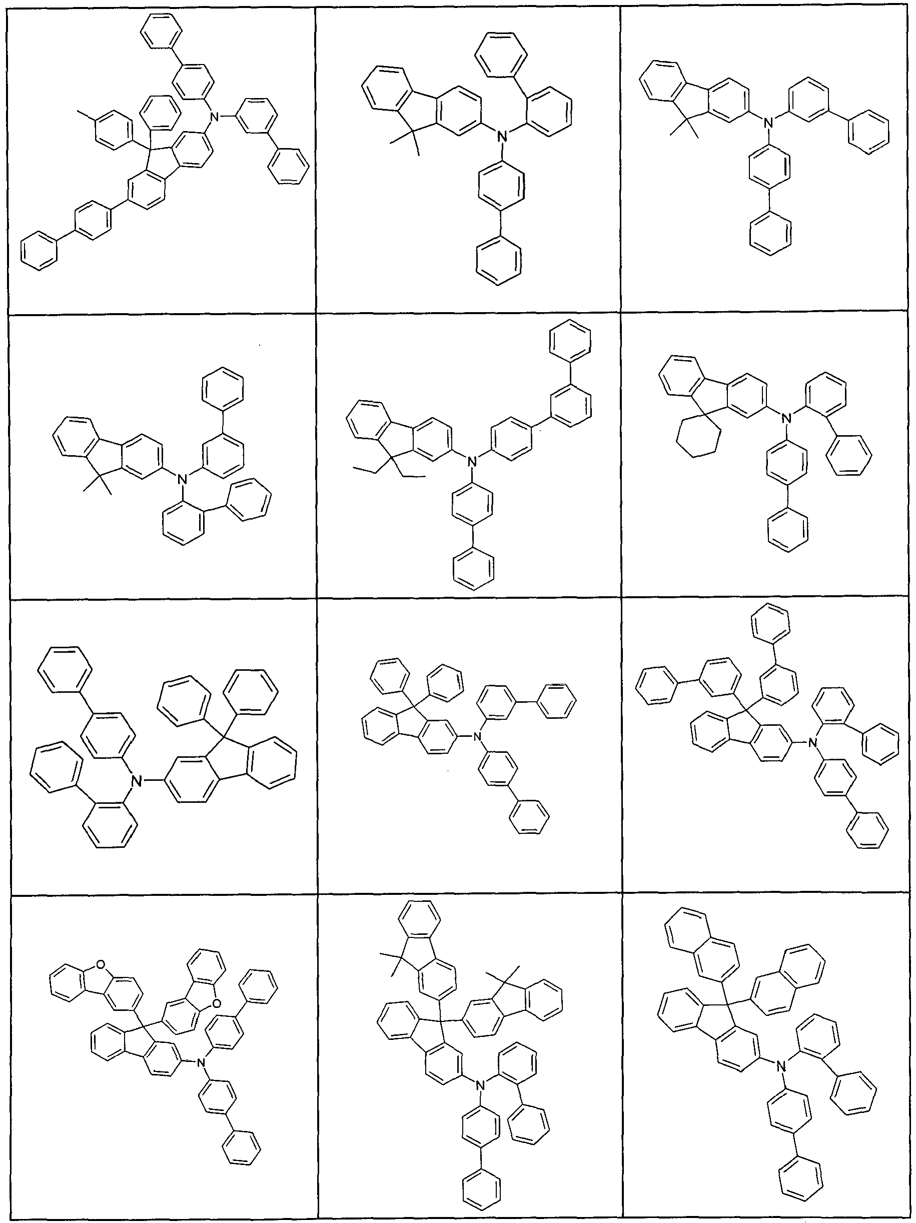

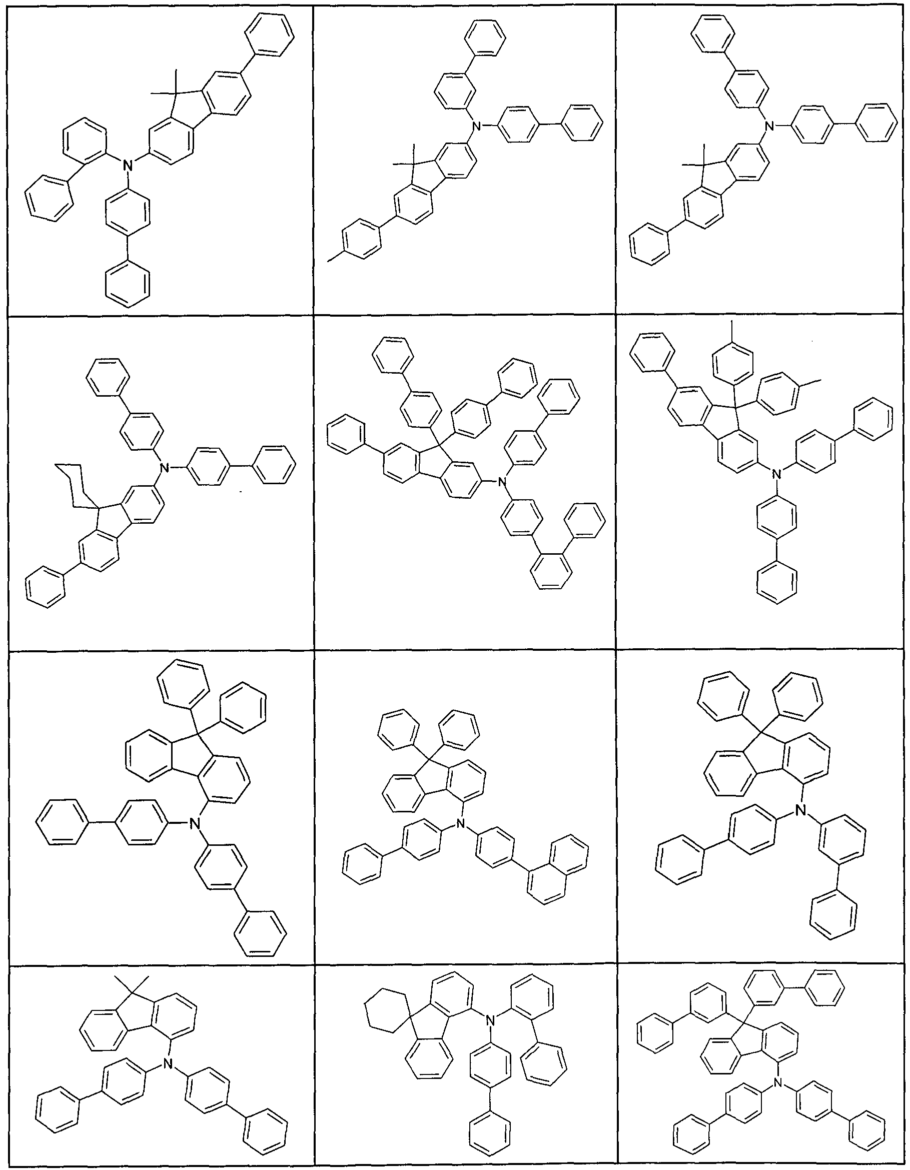

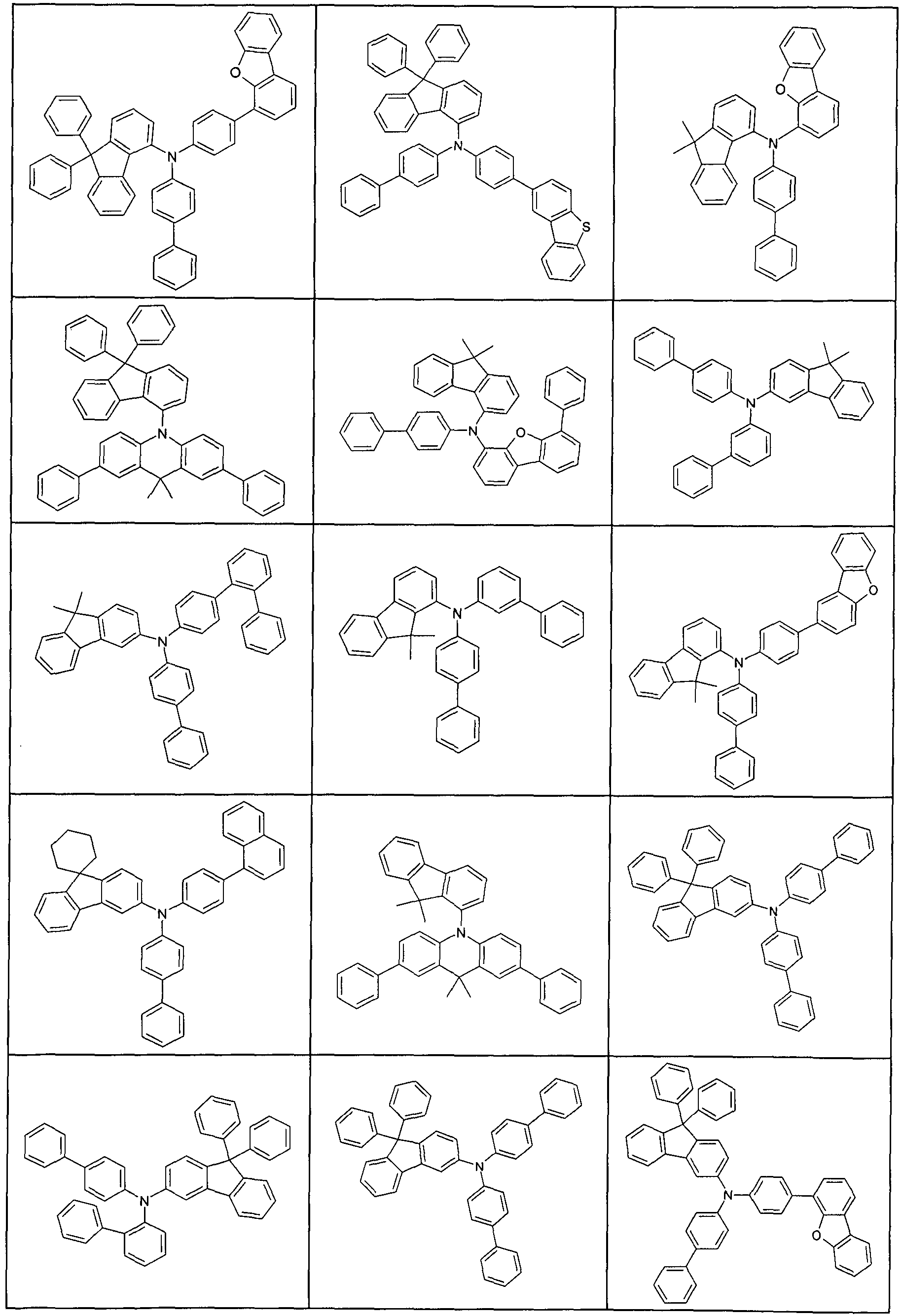

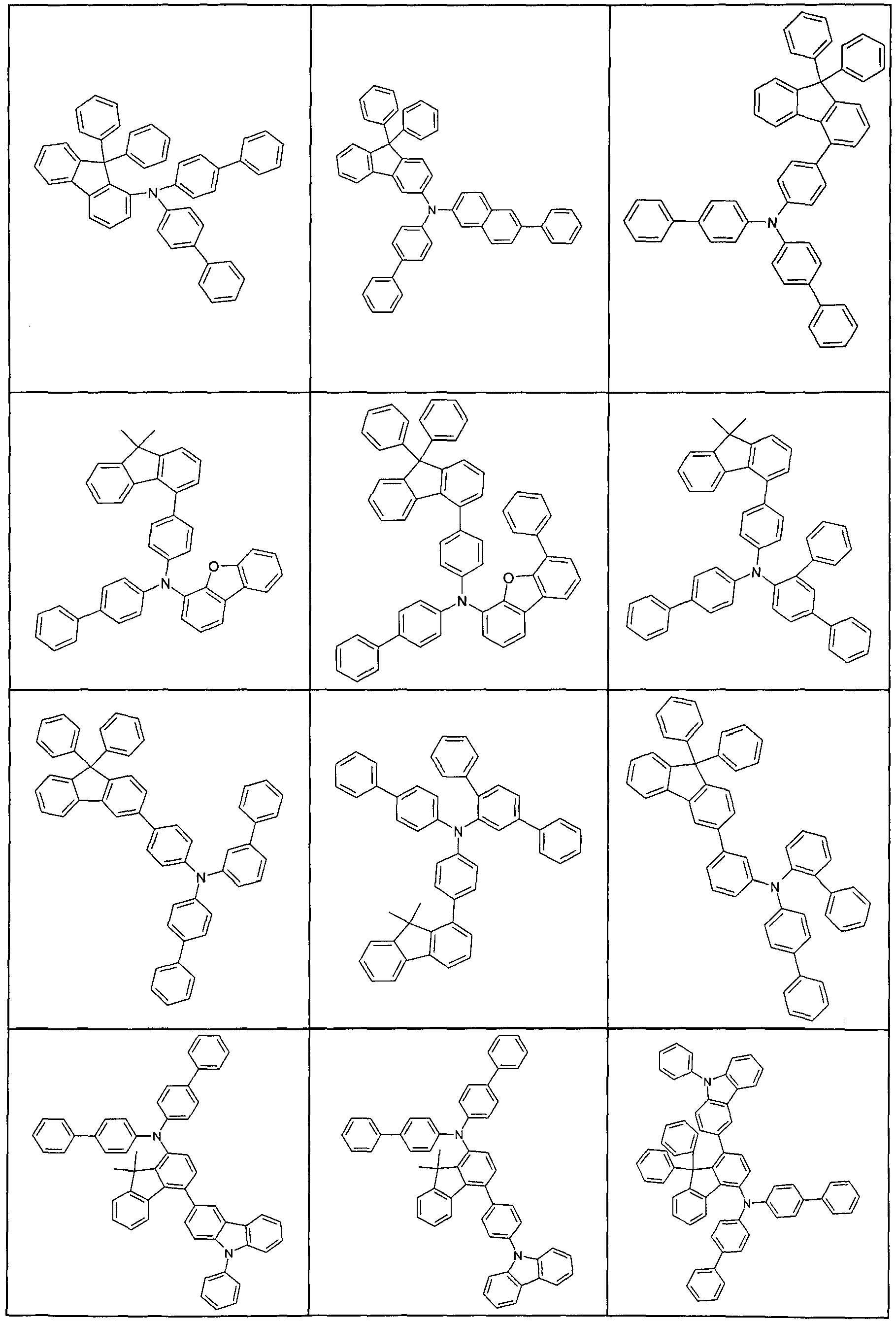

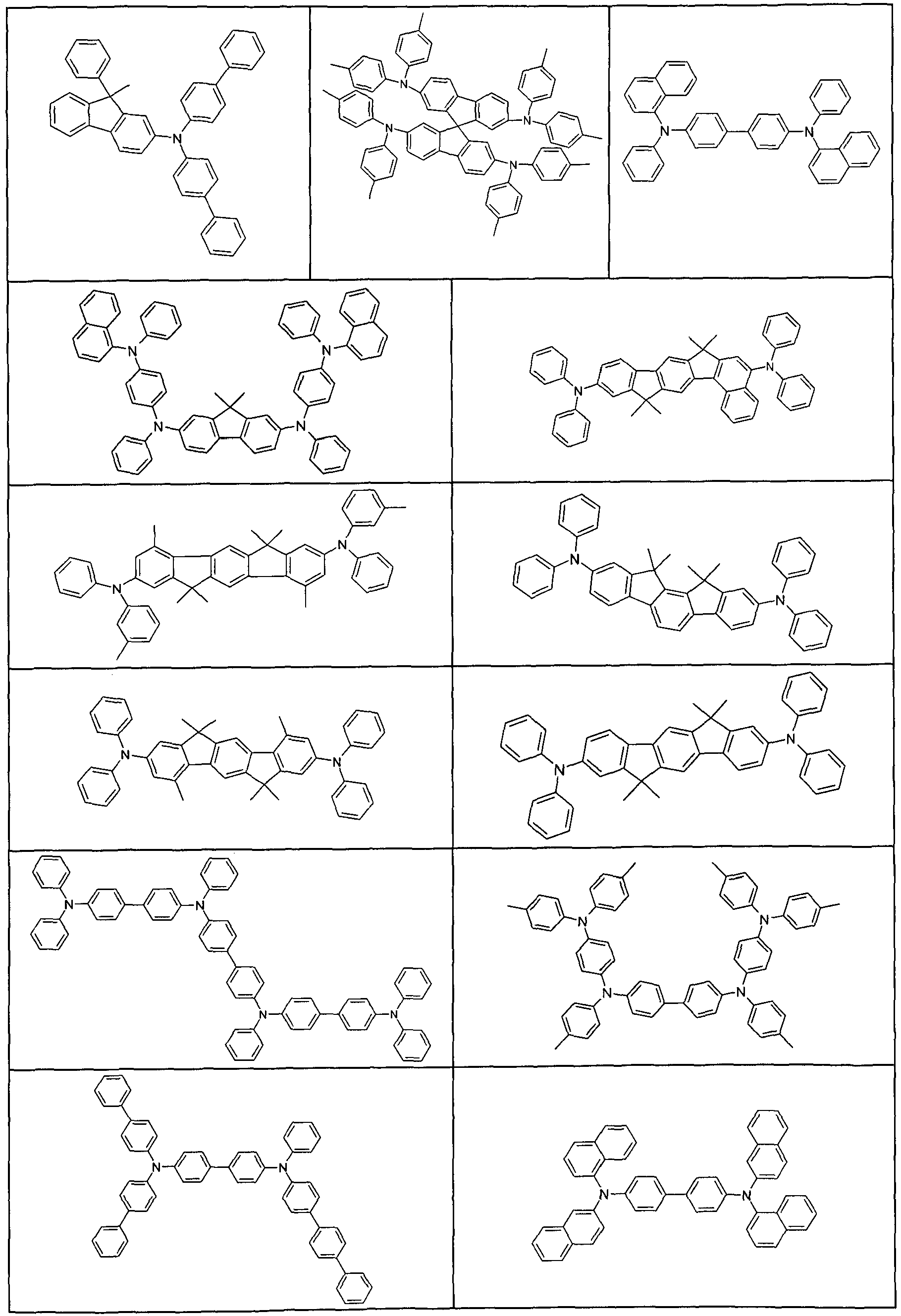

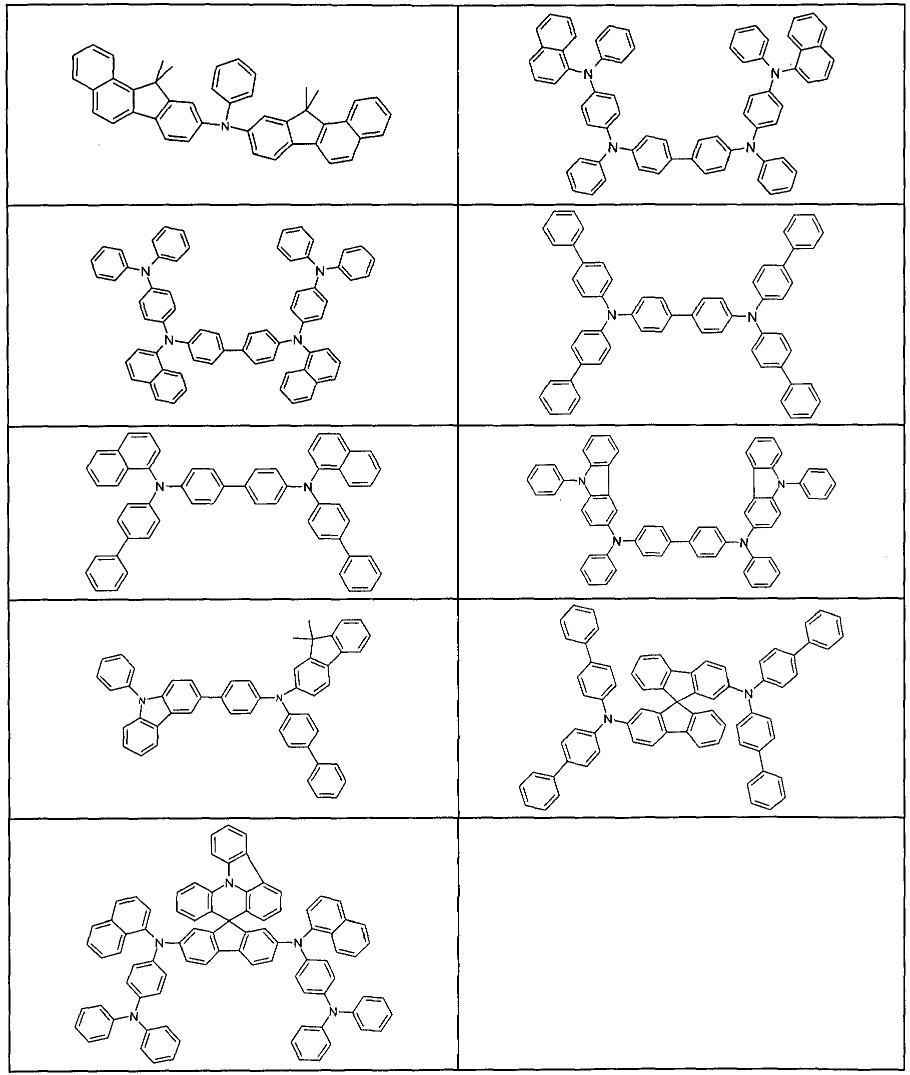

Gemäß der Erfindung ist die Aminverbindung ein Polymer.

Die Polymere enthaltend Triarylamingruppen umfassen mindestens eine Struktureinheit, die der folgenden Formel (I) entspricht 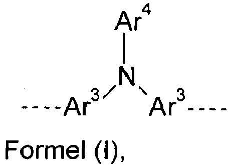 wobei gilt:
- Ar³ , Ar⁴: ist bei jedem Auftreten gleich oder verschieden ein aromatisches Ringsystem mit 6 bis 40 aromatischen Ringatomen, das mit einem oder mehreren Resten R³ substituiert sein kann, oder ein heteroaromatisches Ringsystem mit 5 bis 40 aromatischen Ringatomen, das mit einem oder mehreren Resten R³ substituiert sein kann;
- R³: ist bei jedem Auftreten gleich oder verschieden gewählt aus H, D, F, Cl, Br, I, B(OR⁴)₂, C(=O)R⁴, CN, Si(R⁴)₃, N(R⁴)2, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch-R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, Cl, Br, I, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können; und
die gestrichelten Linien Bindungen zu benachbarten Struktureinheiten im Polymer darstellen.

Unter dem Begriff "Struktureinheit" wird in der vorliegenden Anmeldung eine Einheit verstanden, die, ausgehend von einer Monomereinheit, die mindestens zwei, vorzugsweise zwei, reaktive Gruppen aufweist, durch Reaktion unter Verbindungsknüpfung als ein Teil des Polymergrundgerüstes in dieses eingebaut wird, und damit verknüpft im hergestellten Polymer als Wiederholungseinheit vorliegt.

Die erfindungsgemäßen polymeren Verbindungen weisen vorzugsweise 10 bis 10000, besonders bevorzugt 10 bis 5000 und ganz besonders bevorzugt 10 bis 2000 Struktureinheiten (d.h. Wiederholungseinheiten) auf. Die erfindungsgemäßen oligomeren Verbindungen weisen vorzugsweise 3 bis 9 Struktureinheiten auf. Der Verzweigungs-Faktor der Polymere liegt dabei zwischen 0 (lineares Polymer, ohne Verzweigungsstellen) und 1 (vollständig verzweigtes Dendrimer).

Die erfindungsgemäß einsetzbaren Polymere weisen vorzugsweise ein Molekulargewicht M_{w} im Bereich von 1.000 bis 2.000.000 g/mol, besonders bevorzugt ein Molekulargewicht M_{w} im Bereich von 10.000 bis 1.500.000 g/mol und ganz besonders bevorzugt ein Molekulargewicht M_{w} im Bereich von 50.000 bis 1.000.000 g/mol auf. Die Bestimmung des Molekulargewichts M_{w} erfolgt mittels GPC (= Gelpermeationschromatographie) gegen einen internen Polystyrolstandard.

Bei den erfindungsgemäßen Polymeren handelt es sich entweder um konjugierte, teilkonjugierte oder nicht-konjugierte Polymere. Bevorzugt sind konjugierte oder teilkonjugierte Polymere.

Die Struktureinheiten der Formel (I) können erfindungsgemäß in die Haupt-oder in die Seitenkette des Polymers eingebaut werden. Vorzugsweise werden die Struktureinheiten der Formel (I) jedoch in die Hauptkette des Polymers eingebaut. Bei Einbau in die Seitenkette des Polymeren können die Struktureinheiten der Formel (I) entweder mono- oder bivalent sein, d.h. sie weisen entweder eine oder zwei Bindungen zu benachbarten Struktureinheiten im Polymer auf.

"Konjugierte Polymere" im Sinne der vorliegenden Anmeldung sind Polymere, die in der Hauptkette hauptsächlich sp²-hybridisierte (bzw. gegebenenfalls auch sp-hybridisierte) Kohlenstoffatome enthalten, die auch durch entsprechend hybridisierte Heteroatome ersetzt sein können. Dies bedeutet im einfachsten Fall abwechselndes Vorliegen von Doppel-und Einfachbindungen in der Hauptkette, aber auch Polymere mit Einheiten wie beispielsweise einem meta-verknüpften Phenylen sollen im Sinne dieser Anmeldung als konjugierte Polymere gelten. "Hauptsächlich" meint, dass natürlich (unwillkürlich) auftretende Defekte, die zu Konjugationsunterbrechungen führen, den Begriff "konjugiertes Polymer" nicht entwerten. Als konjugierte Polymere gelten ebenfalls Polymere mit einer konjugierten Hauptkette und nicht-konjugierten Seitenketten. Des Weiteren wird in der vorliegenden Anmeldung ebenfalls als konjugiert bezeichnet, wenn sich in der Hauptkette beispielsweise Arylamineinheiten, Arylphosphineinheiten, bestimmte Heterocyclen (d.h. Konjugation über N-, O- oder S-Atome) und/oder metallorganische Komplexe (d.h. Konjugation über das Metallatom) befinden. Analoges gilt für konjugierte Dendrimere. Hingegen werden Einheiten wie beispielsweise einfache Alkylbrücken, (Thio)Ether-, Ester-, Amid- oder Imidverknüpfungen eindeutig als nicht-konjugierte Segmente definiert.

Unter einem teilkonjugierten Polymer soll in der vorliegenden Anmeldung ein Polymer verstanden werden, das konjugierte Regionen enthält, die durch nicht-konjugierte Abschnitte, gezielte Konjugationsunterbrecher (z.B. Abstandsgruppen) oder Verzweigungen voneinander getrennt sind, z.B. in dem längere konjugierte Abschnitte in der Hauptkette durch nicht-konjugierte Abschnitte unterbrochen sind, bzw. das längere konjugierte Abschnitte in den Seitenketten eines in der Hauptkette nicht-konjugierten Polymers enthält. Konjugierte und teilkonjugierte Polymere können auch konjugierte, teilkonjugierte oder nicht-konjugierte Dendrimere enthalten.

Unter dem Begriff "Dendrimer" soll in der vorliegenden Anmeldung eine hochverzweigte Verbindung verstanden werden, die aus einem multifunktionellen Zentrum (core) aufgebaut ist, an das in einem regelmäßigen Aufbau verzweigte Monomere gebunden werden, so dass eine baumartige Struktur erhalten wird. Dabei können sowohl das Zentrum als auch die Monomere beliebige verzweigte Strukturen annehmen, die sowohl aus rein organischen Einheiten als auch Organometallverbindungen oder Koordinationsverbindungen bestehen. "Dendrimer" soll hier allgemein so verstanden werden, wie dies z.B. von M. Fischer und F. Vögtle (Angew. Chem., Int. Ed. 1999, 38, 885) beschrieben ist.

Bevorzugte Gruppen Ar⁴ in Formel (I) sind die folgenden:

| | | |
|---|---|---|
| 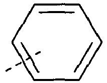 | 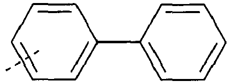 | 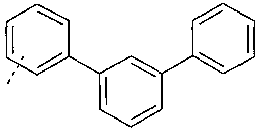 |
| E1 | E2 | E3 |
| 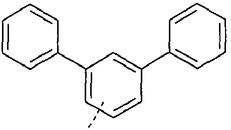 | 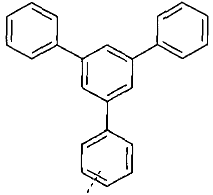 | 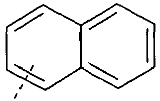 |
| E4 | E5 | E6 |
| 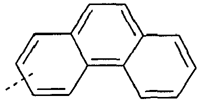 | 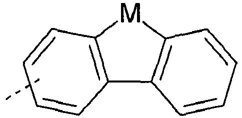 | 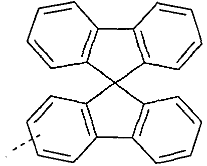 |
| E7 | E8 | E9 |
| 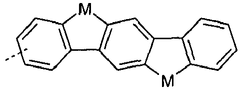 | 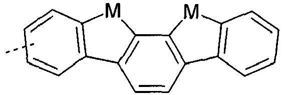 | 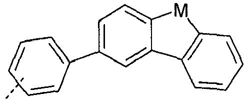 |
| E10 | E11 | E12 |

wobei die Gruppen an allen freien Positionen wahlweise mit Resten R³ substituiert sein können, wobei M bei jedem Auftreten gleich oder verschieden C(R³)₂, NR³, Si(R³)₂, O, S, C=O oder P=O ist, vorzugsweise C(R³)₂, Si(R³)₂, NR, O oder S, und wobei die gestrichelte Linie die Bindungsstelle an die benachbarte Gruppe darstellt.

Bevorzugte mono- oder polycyclische, aromatische oder heteroaromatische Gruppen Ar³ in Formel (I) sind die folgenden:

| | | |
|---|---|---|
| 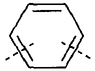 | 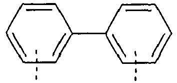 | 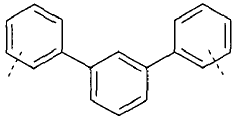 |
| M1 | M2 | M3 |
| 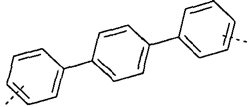 | 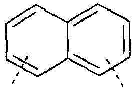 | 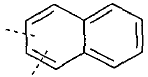 |
| M4 | M5 | M6 |
| 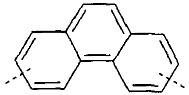 | 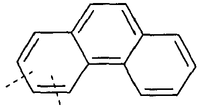 | 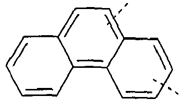 |
| M7 | M8 | M9 |
| 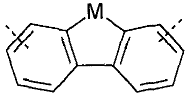 | 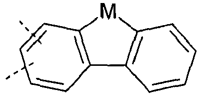 | 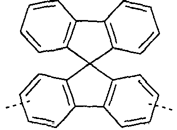 |
| M10 | M11 | M12 |
| 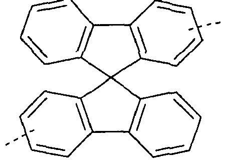 | 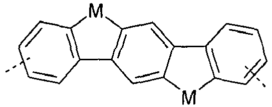 | 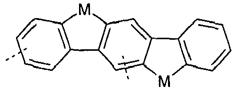 |
| M13 | M14 | M15 |
| 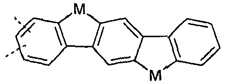 | 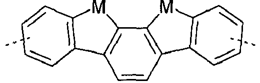 | 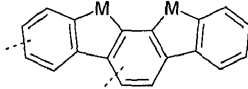 |
| M16 | M17 | M18 |
| 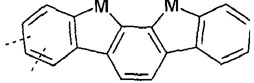 | 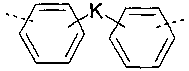 | 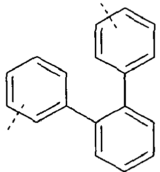 |
| M19 | M20 | M21 |
| 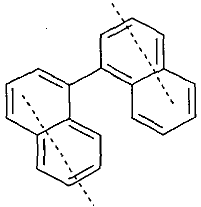 | 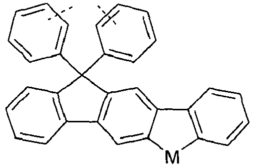 | |
| M22 | M23 | |

wobei die Gruppen an allen freien Positionen wahlweise mit Resten R³ substituiert sein können,
wobei M bei jedem Auftreten gleich oder verschieden C(R³)₂, NR³, Si(R³)₂, O, S, C=O oder P=O ist, vorzugsweise C(R³)₂, Si(R³)₂, NR, O oder S,
wobei K C(R³)₂, Si(R³)₂, NR³, O, S, eine geradkettige Alkyl- oder Alkoxygruppe mit 1 bis 20 C-Atomen, eine verzweigte oder cyclische Alkyl-oder Alkoxygruppe mit 3 bis 20 C-Atomen, eine Alkenyl- oder Alkinylgruppe mit 2 bis 20 C-Atomen, ein aromatisches Ringsystem mit 6 bis 40 aromatischen Ringatomen, oder ein heteroaromatisches Ringsystem mit 5 bis 40 aromatischen Ringatomen ist; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl-und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, und
wobei die gestrichelten Linien die Bindungsstellen an die benachbarten Gruppen darstellen.

Es ist erfindungsgemäß bevorzugt, dass mindestens eine der Struktureinheiten der Formel (I) mindestens eine vernetzbare Gruppe Q aufweist.

"Vernetzbare Gruppe Q" im Sinne der vorliegenden Erfindung bedeutet eine funktionelle Gruppe, die in der Lage ist, eine Reaktion einzugehen und so eine unlösliche Verbindung zu bilden. Die Reaktion kann dabei mit einer weiteren, gleichen Gruppe Q, einer weiteren, verschiedenen Gruppe Q oder einem beliebigen anderen Teil derselben oder einer anderen Polymerkette erfolgen. Bei der vernetzbaren Gruppe handelt es sich somit um eine reaktive Gruppe. Dabei erhält man als Ergebnis der Reaktion der vernetzbaren Gruppe eine entsprechend vernetzte Verbindung. Die chemische Reaktion kann auch in der Schicht durchgeführt werden, wobei eine unlösliche Schicht entsteht. Die Vernetzung kann gewöhnlich durch Wärme oder durch UV-, Mikrowellen-, Röntgen- oder Elektronenstrahlung unterstützt werden, gegebenenfalls in Gegenwart eines Initiators. "Unlöslich" im Sinne der vorliegenden Erfindung bedeutet vorzugsweise, dass das erfindungsgemäße Polymer nach der Vernetzungsreaktion, also nach der Reaktion der vernetzbaren Gruppen, bei Raumtemperatur in einem organischen Lösungsmittel eine Löslichkeit aufweist, die mindestens einen Faktor 3, vorzugsweise mindestens einen Faktor 10, geringer ist als die des entsprechenden, nicht-vernetzten, erfindungsgemäßen Polymers in demselben organischen Lösungsmittel.

Mindestens eine vernetzbare Gruppe heißt in der vorliegenden Anmeldung, dass eine Struktureinheit eine oder mehrere vernetzbare Gruppen aufweist. Vorzugsweise weist eine Struktureinheit genau eine vernetzbare Gruppe auf.

Weist die Struktureinheit der Formel (I) eine vernetzbare Gruppe auf, so kann diese an Ar³ oder Ar⁴ gebunden sein. Vorzugsweise ist die vernetzbare Gruppe an Ar⁴ gebunden.

Als Gruppen Q können generell alle Gruppen eingesetzt werden, die dem Fachmann für diesen Zweck bekannt sind. Es ist insbesondere die Aufgabe dieser Gruppe, durch eine Vernetzungsreaktion die erfindungsgemäßen polymeren Verbindungen, gegebenenfalls mit weiteren reaktiven polymeren Verbindungen, miteinander zu verknüpfen. Dies führt zu einer vernetzten Verbindung, bzw., wenn die Reaktion in einer Schicht durchgeführt wird, zu einer vernetzten Schicht. Unter einer vernetzten Schicht im Sinne der vorliegenden Erfindung wird eine Schicht verstanden, die erhältlich ist durch Durchführung der Vernetzungsreaktion aus einer Schicht der erfindungsgemäßen, vernetzbaren, polymeren Verbindung. Die Vernetzungsreaktion kann im Allgemeinen durch Wärme und/oder durch UV-, Mikrowellen-, Röntgen- oder Elektronenstrahlung und/oder durch den Einsatz von Radikalbildnern, Anionen, Kationen, Säuren und/oder Photosäuren initiiert werden. Ebenso kann die Anwesenheit von Katalysatoren sinnvoll oder notwendig sein. Bevorzugt ist die Vernetzungsreaktion eine Reaktion, für die kein Initiator und kein Katalysator zugesetzt werden muss.

Erfindungsgemäß bevorzugte vernetzbare Gruppen Q sind die im Folgenden aufgeführten Gruppen:
a) Endständige oder cyclische Alkenyl- bzw. endständige Dienyl- und Alkinylgruppen:
   Geeignet sind Einheiten, die eine endständige oder cyclische Doppelbindung, eine endständige Dienylgruppe oder eine endständige Dreifachbindung enthalten, insbesondere endständige oder cyclische Alkenyl-, endständige Dienyl- bzw. endständige Alkinylgruppen mit 2 bis 40 C-Atomen, vorzugsweise mit 2 bis 10 C-Atomen, wobei auch einzelne CH₂-Gruppen und/oder einzelne H-Atome durch die oben genannten Gruppen R ersetzt sein können. Weiterhin eignen sich auch Gruppen, die als Vorstufen zu betrachten sind und die in situ zu einer Bildung einer Doppel- oder Dreifachbindung in der Lage sind.
b) Alkenyloxy-, Dienyloxy- bzw. Alkinyloxygruppen:
   Weiterhin geeignet sind Alkenyloxy-, Dienyloxy- bzw. Alkinyloxygruppen, vorzugsweise Alkenyloxygruppen.
c) Acrylsäuregruppen:
   Weiterhin geeignet sind Acrylsäureeinheiten im weitesten Sinne, vorzugsweise Acrylester, Acrylamide, Methacrylester und Methacrylamide. Besonders bevorzugt sind C₁₋₁₀-Alkylacrylat und C₁₋₁₀-Alkylmethacrylat.
   Die Vernetzungsreaktion der oben unter a) bis c) genannten Gruppen kann über einen radikalischen, einen kationischen oder einen anionischen Mechanismus aber auch über Cycloaddition erfolgen.
   Es kann sinnvoll sein, einen entsprechenden Initiator für die Vernetzungsreaktion zuzugeben. Geeignete Initiatoren für die radikalische Vernetzung sind beispielsweise Dibenzoylperoxid, AIBN oder TEMPO. Geeignete Initiatoren für die kationische Vernetzung sind beispielsweise AlCl₃, BF₃, Triphenylmethylperchlorat oder Tropyliumhexachlorantimonat. Geeignete Initiatoren für die anionische Vernetzung sind Basen, insbesondere Butyllithium.
   In einer bevorzugten Ausführungsform der vorliegenden Erfindung wird die Vernetzung jedoch ohne den Zusatz eines Initiators durchgeführt und ausschließlich thermisch initiiert. Diese Bevorzugung wird dadurch begründet, dass die Abwesenheit des Initiators Verunreinigungen der Schicht verhindert, die zu einer Verschlechterung der Deviceeigenschaften führen könnten.
d) Oxetane und Oxirane:
   Eine weitere geeignete Klasse vernetzbarer Gruppen Q sind Oxetane und Oxirane, die durch Ringöffnung kationisch vernetzen.
   Es kann sinnvoll sein, einen entsprechenden Initiator für die Vernetzungs-reaktion zuzugeben. Geeignete Initiatoren sind beispielsweise AlCl₃, BF₃, Triphenylmethylperchlorat oder Tropyliumhexachlorantimonat. Ebenso können Photosäuren als Initiatoren zugegeben werden.
e) Silane:
   Weiterhin geeignet als Klasse vernetzbarer Gruppen sind Silangruppen SiR₃, wobei mindestens zwei Gruppen R, bevorzugt alle drei Gruppen R für Cl oder eine Alkoxygruppe mit 1 bis 20 C-Atomen stehen.
   Diese Gruppe reagiert in der Anwesenheit von Wasser zu einem Oligo-oder Polysiloxan.
f) Cyclobutangruppen
   Die oben genannten vernetzbaren Gruppen Q sind dem Fachmann generell bekannt, ebenso wie die geeigneten Reaktionsbedingungen, die zur Reaktion dieser Gruppen verwendet werden.

Der Anteil an Struktureinheiten der Formeln (I) im Polymer liegt im Bereich von 1 bis 100 mol%, vorzugsweise im Bereich von 25 bis 100 mol%, besonders bevorzugt im Bereich von 50 bis 95 mol%, bezogen auf 100 mol% aller copolymerisierten Monomere, die im Polymer als Struktureinheiten enthalten sind.

Das Polymer enthält eine weitere Struktureinheit der folgenden Formel (II), die von der Struktureinheit der Formel (I) verschieden ist:

---- Ar⁵---- Formel (II)

wobei
Ar⁵ gewählt ist aus Indenofluorenen.

Besonders bevorzugte Amin-Polymere zur Verwendung als Aminverbindung in der aus Lösung aufgebrachten Schicht gemäß der vorliegenden Erfindung sind im Folgenden aufgeführt:

| |
|---|
| 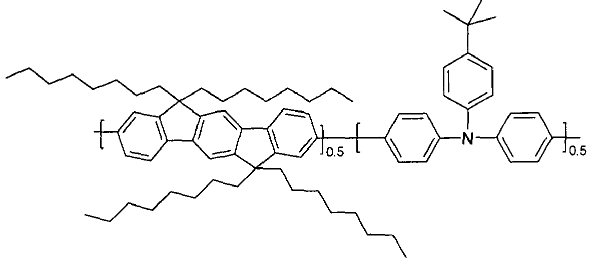 |
| P-1 |
| |
| 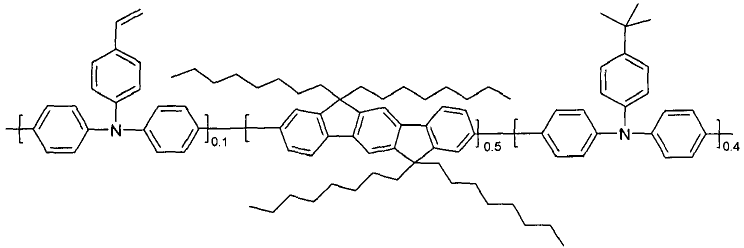 |
| P-2 |
| |
| 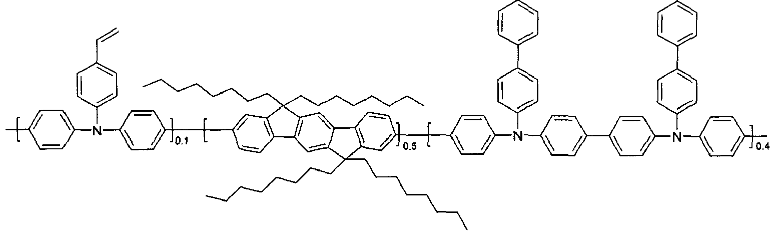 |
| P-3 |

Bevorzugt ist das Amin-Polymer zur Verwendung als Aminverbindung in der aus Lösung aufgebrachten Schicht nicht das oben genannte P-3.

Die erfindungsgemäß einsetzbaren Polymere werden in der Regel durch Polymerisation von einer oder mehreren Monomersorten hergestellt, von denen mindestens ein Monomer im Polymer zu Struktureinheiten der Formel (I) und/oder (II) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation;
(D) HECK-Polymerisation;
(E) NEGISHI-Polymerisation;
(F) SONOGASHIRA-Polymerisation;
(G) HIYAMA-Polymerisation; und
(H) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in der WO 03/048225 A2, der WO 2004/037887 A2 und der WO 2004/037887 A2 im Detail beschrieben. Die C-C-Verknüpfungen sind vorzugsweise ausgewählt aus den Gruppen der SUZUKI-Kupplung, der YAMAMOTO-Kupplung und der STILLE-Kupplung; die C-N-Verknüpfung ist vorzugsweise eine Kupplung gemäß HARTWIG-BUCHWALD.

Erfindungsgemäß wird die zwischen Anode und emittierender Schicht angeordnete Schicht, welche eine Aminverbindung enthält, aus Lösung aufgebracht. Das Aufbringen aus Lösung erfolgt bevorzugt durch Spincoating, oder mit einem beliebigen Druckverfahren, wie z. B. Siebdruck, Flexodruck, Nozzle Printing oder Offsetdruck, besonders bevorzugt aber durch LITI (Light Induced Thermal Imaging, Thermotransferdruck) oder Ink-Jet Druck (Tintenstrahldruck).

Hierzu wird eine Formulierung, enthaltend die Aminverbindung und wenigstens ein Lösungsmittel benötigt.

Zur Herstellung der Formulierung werden die Aminverbindung und gegebenfalls weitere in der Schicht enthaltende Verbindungen in einem geeigneten Lösungsmittel gelöst.

Bevorzugt werden hierzu die einzelnen Komponenten der Formulierung gemischt und gerührt, gegebenenfalls auch unter Zufuhr von Wärme. Häufig wird die Formulierung auch entgast oder mit Inertgasen übersättigten Lösungsmitteln hergestellt. Insgesamt ist darauf zu achten, dass nur Lösungsmittel und andere Komponenten sehr hoher Reinheit verwendet werden, um eine Kontamination der elektronischen Vorrichtungen mit schädlichen Verbindungen zu vermeiden. Insbesondere ist darauf zu achten, dass der Wasser-, Sauerstoff und Halogengehalt in der Formulierung niedrig gehalten wird, da insbesondere die Leistungsdaten organischer Elektrolumineszenzvorrichtungen durch deren Anwesenheit stark beeinträchtigt werden können.

In der verwendeten Formulierung können ein einziges oder mehrere Lösungsmittel enthalten sein.

Die Lösungsmittel sind erfindungsgemäß bevorzugt gewählt aus Lösungsmitteln mit einer Oberflächenspannung von wenigstens 28 mN/m, bevorzugt wenigstens 30 mN/m, ganz bevorzugt wenigstens 32 mN/m und ganz besonders bevorzugt wenigstens 35 mN/m.

Weiterhin bevorzugt ist, wenn die Siede- oder Sublimationstemperatur der verwendeten Lösungsmittel weniger als 300°C und bevorzugt weniger als 260°C beträgt.

Ganz bevorzugt ist, wenn die Viskosität der Lösungsmittel größer als 3mPa*s und bevorzugt größer als 5mPa*s ist.

Weiterhin bevorzugt ist, wenn das Molekulargewicht der Lösungsmittel kleiner oder gleich 1000 g/mol, bevorzugt kleiner oder gleich 700 g/mol, ganz bevorzugt kleiner oder gleich 500 g/mol und besonders bevorzugt kleiner oder gleich 300 g/mol ist.

Bevorzugt ist die Konzentration der Aminverbindung in der Formulierung bezogen auf die Gesamtformulierung im Bereich von 0.5 bis 20 Gew.-%, besonders bevorzugt im Bereich von 1 bis 15 Gew.-% und ganz besonders bevorzugt im Bereich von 1.5 bis 10 Gew.-%. Für das Aufbringen der Formulierung mittels Spin-Coating ist es bevorzugt, dass die Konzentration der Aminverbindung in der Formulierung bezogen auf die Gesamtformulierung im Bereich von 0.5 bis 5 Gew.-% liegt, besonders bevorzugt im Bereich von 1 bis 4 Gew.-%.

Bevorzugte Lösungsmittel sind gewählt aus aromatischen Lösungsmitteln. Besonders bevorzugte Lösungsmittel sind gewählt aus aromatischen Kohlenwasserstoffen, wie Toluol, o-, m- oder p-Xylol, Phenoxytoluol, Trimethylbenzolen (z.B. 1,2,3-, 1,2,4- und 1,3,5-Trimethylbenzol), Tetralin, anderen Mono-, Di-, Tri- und Tetraalkylbenzolen (z.B. Diethylbenzol, Methylcumol, Tetramethylbenzolen), aromatischen Ethern (z.B. Anisol, Alkylanisolen, z.B. 2-, 3- und 4-Isomere des Methylanisols, 2,3-, 2,4-, 2,5-, 2,6-, 3,4- und 3,5-Isomere des Dimethylanisols), Naphthalinderivaten, Alkylnaphthalinderivaten (z.B. 1- und 2- Methylnaphthalin), und Di- und Tetrahydronaphthalinderivaten. Ebenfalls bevorzugt sind aromatische Ester (z.B. Alkylbenzoate), aromatische Ketone (z.B. Acetophenon, Propiophenon), Alkylketone (z.B. Cyclohexanon), heteroaromatische Lösungsmittel (z.B. Thiophen, Mono-, Di- und Trialkylthiophene, 2-Alkylthiazole, Benzthiazole usw., Pyridine), Halogenarylene und Anilinderivate. Diese Lösungsmittel können Halogenatome enthalten.

Besonders bevorzugt sind als Lösungsmittel aromatische Kohlenwasserstoffe, insbesondere Toluol, Phenoxytoluol, Dimethylbenzole (Xylole), Trimethylbenzole, Tetralin und Methylnaphthaline, aromatische Ether, insbesondere Anisol, und aromatische Ester, insbesondere Methylbenzoat. Noch stärker bevorzugt sind aromatische Ether, insbesondere Anisol und Derivate davon, wie Alkylanisole, und aromatische Ester, insbesondere Methylbenzoat.

Explizite Beispiele für bevorzugte Lösungsmittel sind Toluol, Anisol, o-, m-oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethyhenglycolbutylmethylether, Triethyleneglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Die erfindungsgemäße Vorrichtung enthält bevorzugt zusätzlich zu Anode, Kathode, emittierender Schicht, und mindestens einer zwischen Anode und emittierender Schicht angeordneten Schicht noch zusätzliche Schichten. Diese zusätzlichen Schichten sind bevorzugt gewählt aus Pufferschichten, Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen. Die Funktion und bevorzugte Anordnung dieser Schichten sind dem Fachmann bekannt. Weiterhin sind dem Fachmann mögliche Verbindungen zur Verwendung in den jeweiligen Schichten bekannt.

Die Schichten der erfindungsgemäßen Vorrichtung enthalten bevorzugt eine oder mehrere organische Verbindungen. Besonders bevorzugt sind sie im Wesentlichen aus organischen Verbindungen aufgebaut, sind also organische Schichten.

In den Schichten können alle Materialien verwendet werden, wie sie üblicherweise gemäß dem Stand der Technik in den betreffenden Schichten eingesetzt werden.

Die Abfolge der Schichten der erfindungsgemäßen Vorrichtung ist bevorzugt wie folgt:
- Anode
- optionale Pufferschicht (buffer layer)
- Schicht, welche eine Aminverbindung enthält, und welche aus Lösung aufgebracht ist
- optional weitere Lochtransportschichten
- optional Elektronenblockierschicht
- emittierende Schicht
- optional Lochblockierschicht
- Elektronentransportschicht
- optional weitere Elektronentransportschichten
- optional Elektroneninjektionsschicht
- Kathode.

Es können zusätzlich weitere Schichten in der erfindungsgemäßen Vorrichtung vorhanden sein.

Bevorzugt umfasst die erfindungsgemäße Vorrichtung nicht die folgende Schichtabfolge:
- Substrat
- Anode aus ITO
- Pufferschicht enthaltend PEDOT:PSS
- HTL enthaltend das oben genannte Polymer P-3, aus Lösung aufgebracht
- Emittierende Schicht enthaltend die Verbindungen 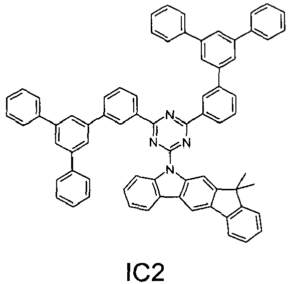 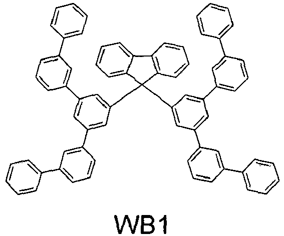 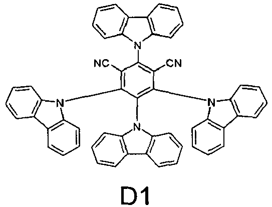
- Elektronentransportschicht, enthaltend die Verbindung ST1 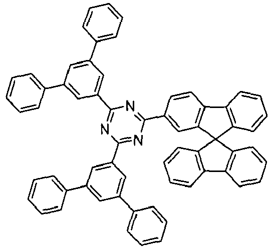 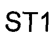
- Elektroneninjektionsschicht, enthaltend die Verbindungen ST1 und LiQ 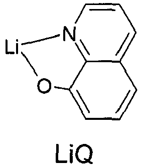
- Kathode aus Aluminium.

Bevorzugt enthält die erfindungsgemäße Vorrichtung eine Pufferschicht zwischen Anode und der Schicht, welche aus Lösung aufgebracht ist. Diese Pufferschicht enthält bevorzugt ein leitfähiges organisches Polymer, besonders bevorzugt PEDOT:PSS oder PANI (Polyanilin). Hierdurch wird die Lochinjektion und damit die Leistungsdaten der OLED verbessert. Weiterhin wird dadurch die Zuverlässigkeit erhöht, insbesondere die Ausfallhäufigkeit verringert.

Gemäß einer alternativen, unter bestimmten Umständen ebenfalls bevorzugten Ausführungsform ist die Schicht, welche eine Aminverbindung enthält und aus Lösung aufgebracht ist, direkt, d.h. ohne dazwischenliegende Schicht, auf die Anode aufgebracht. Hierdurch wird in besonderem Maß der Vorteil einer geglätteten Oberfläche der Schicht, welche eine Aminverbindung enthält, erhalten.

Bevorzugt umfasst die erfindungsgemäße Vorrichtung genau eine emittierende Schicht. Sie kann jedoch auch mehrere emittierende Schichten enthalten, was insbesondere von Interesse ist, wenn die Vorrichtung als Beleuchtungsvorrichtung genutzt werden soll. Wenn die erfindungsgemäße Vorrichtung mehrere emittierende Schichten enthält, ist die aus Lösung aufgetragene Schicht enthaltend eine Aminverbindung bevorzugt zwischen Anode und der anodennächsten der mehreren emittierenden Schichten angeordnet.

Gemäß einer bevorzugten Ausführungsform sind die Lochtransportschichten p-dotiert und/oder die Elektronentransportschichten sind n-dotiert. Unter einer p-dotierten Schicht wird eine Schicht verstanden, in der durch eine in geringer Menge vorhandene Verbindung (einen p-Dotanden) freie Löcher erzeugt werden und deren Leitfähigkeit dadurch erhöht ist. Unter einer n-dotierten Schicht wird eine Schicht verstanden, in der durch eine in geringer Menge vorhandene Verbindung (einen n-Dotanden) freie Elektronen erzeugt werden und deren Leitfähigkeit dadurch erhöht ist. Eine umfassende Diskussion von dotierten Transportschichten in OLEDs findet sich in Chem. Rev. 2007, 107, 1233. Besonders bevorzugt ist der p-Dotand in der Lage, das Lochtransportmaterial in der Lochtransportschicht zu oxidieren, hat also ein ausreichend hohes Redoxpotential, insbesondere ein höheres Redoxpotential als das Lochtransportmaterial. Als p-Dotanden sind prinzipiell alle Verbindungen geeignet, welche Elektronenakzeptorverbindungen darstellen und die Leitfähigkeit der organischen Schicht durch Oxidation des Lochtransportmaterials erhöhen können. Der Fachmann kann im Rahmen seines allgemeinen Fachwissens ohne größeren Aufwand geeignete Verbindungen identifizieren. Entsprechendes gilt mutatis mutandis für n-Dotanden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Bevorzugt wird die organische Elektrolumineszenzvorrichtung in Displays, als Lichtquelle in Beleuchtungsanwendungen sowie als Lichtquelle in medizinischen und/oder kosmetischen Anwendungen (z.B. in der Lichttherapie) eingesetzt werden.

Gegenstand der vorliegenden Anmeldung ist auch ein Verfahren zur Herstellung einer organischen Elektrolumineszenzvorrichtung gemäß Anspruch 10.

Für dieses Verfahren gelten entsprechend die oben angegebenen bevorzugten Ausführungsformen der Vorrichtung.

Beim Aufbringen von Schichten von organischen Elektrolumineszenzvorrichtungen wird zwischen zwei grundsätzlich verschiedenen Verfahren unterscheiden. Beim ersten Verfahren werden die relevanten Verbindungen im Vakuum aufgedampft (Gasphasenabscheidung). Dieses Verfahren ist sehr aufwendig und kostenintensiv. Jedoch sind die Leistungsdaten der mit solchen Verfahren erhaltenen OLEDs typischerweise sehr gut. Bei dem zweiten Verfahren werden die relevanten Verbindungen aus der Lösung aufgebracht, wie dies bei der erfindungsgemäßen Vorrichtung für die zwischen Anode und emittierender Schicht angeordnete Schicht, welche eine Aminoverbindung enthält, der Fall ist. Das lösungsbasierte Verfahren hat das Potential, sehr kosteneffizient zu sein. Zusätzlich ist die Ausfallrate der erhaltenen OLEDs relativ gesehen oftmals niedriger. Jedoch sind die Leistungsdaten der erhaltenen OLEDs oftmals weniger gut als bei OLEDs, bei welchen die Schichten aus Lösung hergestellt wurden. Überraschend wurde jedoch in der vorliegenden Erfindung festgestellt, dass im gegebenen Fall durch die Kombination von lösungsbasierter Auftragung von Schichten und gasphasenbasierter Auftragung von Schichten die Vorteile der beiden Verfahren beide auftreten, während die Nachteile nur vernachlässigbar auftreten. Insbesondere weisen die erhaltenen Vorrichtungen sehr geringe Ausfallraten kombiniert mit sehr guten Leistungsdaten auf.

Gemäß einer bevorzugten Ausführungsform werden außer der zwischen Anode und emittierender Schicht angeordneten Schicht, die aus Lösung aufgetragen wird, eine oder mehrere Schichten der organischen Elektrolumineszenzvorrichtung aus der Gasphase aufgebracht.

Es ist dabei bevorzugt, dass alle Schichten zwischen Anode und emittierender Schicht aus Lösung aufgebracht werden, und dass alle Schichten zwischen emittierender Schicht und Kathode aus der Gasphase aufgebracht werden. Es ist dabei besonders bevorzugt, dass alle Schichten zwischen Anode und emittierender Schicht aus Lösung aufgebracht werden, und dass die emittierende Schicht und alle Schichten zwischen emittierender Schicht und Kathode aus der Gasphase aufgebracht werden. Insbesondere ist es bevorzugt, dass die eine oder mehrere emittierenden Schichten aus der Gasphase aufgebracht sind, insbesondere diejenige emittierende Schicht, welche eine emittierende Verbindung enthält, die eine betragsmäßige Differenz zwischen den Energien ihres S₁- und T₁-Zustands von nicht mehr als 0.15 eV aufweist.

Es ist bevorzugt, dass die emittierende Schicht, welche eine emittierende Verbindung enthält, die eine betragsmäßige Differenz zwischen den Energien ihres S₁- und T₁-Zustands von nicht mehr als 0.15 eV aufweist, nicht aus Toluol-Lösung aufgebracht wird, bevorzugt überhaupt nicht aus Lösung aufgebracht wird.

Werden Schichten aus der Gasphase aufgebracht, so ist es bevorzugt, dass die Materialien in Vakuum-Sublimationsanlagen bei einem Anfangsdruck kleiner 10⁻⁵ mbar, bevorzugt kleiner 10⁻⁶ mbar, aufgedampft werden. Dabei ist es jedoch auch möglich, dass der Anfangsdruck noch geringer ist, beispielsweise kleiner 10⁻⁷ mbar. Alternativen zum oben genannten Sublimationsverfahren sind das OVPD (Organic Vapour Phase Deposition) Verfahren oder die Trägergassublimation.

Die Vorrichtung wird schließlich (je nach Anwendung) strukturiert, kontaktiert und schließlich versiegelt, um schädigende Effekte von Wasser und Luft, insbesondere Sauerstoff, auszuschließen.

Alle oben angegebenen bevorzugten Ausführungsformen betreffend die erfindungsgemäße Vorrichtung gelten, wo anwendbar, auch für das erfindungsgemäße Herstellungsverfahren als bevorzugt.

Die folgenden Beispiele fallen nicht unter den Schutzumfang der Ansprüche, dienen aber der Erläuterung des technischen Hintergrundes.

### A) Herstellung und Charakterisierung von OLEDs

### A-1) Herstellung der OLEDs

In den folgenden Beispielen werden die Daten verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP). Diese Glasplättchen bilden die Substrate, auf welche die OLEDs aufgebracht werden. Nach der UV-Ozon Behandlung werden die Substrate innerhalb 30min weiter verarbeitet.

### A-2) Charakterisierung der OLEDs

Die OLEDs werden standardmäßig charakterisiert. Hierfür werden die Elektrolumineszenzspektren, Spannung und die externe Quanteneffizienz (EQE, gemessen in Prozent) bestimmt. Die EQE wird unter Annahme einer lambertschen Abstrahlcharakteristik aus der Stromeffizienz (in cd/A) in Vorwärtsrichtung berechnet. Die Stromeffizienz wird aus Leuchtdichte und Stromdichte bestimmt. Die Leuchtdichte wird mit einer kalibrierten Photodiode gemessen. Die Elektrolumineszenzspektren werden bei einer Leuchtdichte von 1000 cd/m² bestimmt und daraus die CIE 1931 x und y Farbkoordinaten berechnet. Die Angabe U1000 bezeichnet die Spannung, die für eine Leuchtdichte von 1000 cd/m² benötigt wird. EQE1000 bezeichnet die externe Quanteneffizienz bei einer Betriebsleuchtdichte von 1000 cd/m².

### Referenzbeispiel 1

Das Substrat wird mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert) und anschließend bei 180°C 10min lang ausgeheizt.

Anschließend werden folgende Schichten in der angegebenen Reihenfolge in einer Vakuumkammer thermisch aufgedampft: Eine 15nm dicke Emissionsschicht bestehend zu 85 Vol% aus der Substanz IC1 und zu 15 Vol% aus der Substanz D1, eine 10nm dicke Schicht der Substanz IC1, eine 40nm dicke Schicht der Substanz ST1, eine 3nm dicke Schicht der Substanz LiQ, eine 100nm dicke Schicht Aluminium als Kathode. Anschließend werden die OLEDs verkapselt.

Die Emissionsschicht zeigt eine PLQE von 81 % (Anregungswellenlänge 350nm) und eine Abklingzeit von ta=4.8µs (t_{d}=7µs). Diese Werte gelten auch für Beispiel 2 und die erfindungsgemäßen Beispiele 1-4.

Von diesen OLEDs werden 64 Stück hergestellt. Betreibt man diese bei einer Stromdichte von 20mA/cm², so fallen nach 200h Betrieb sechs (also ca. 9%) davon aus. Die OLEDs zeigen CIE x,y Farbkoordinaten von 0.32/0.56, EQE1000=8.4%, U1000=3.6V.

### Referenzbeispiel 2

Auf das Substrat wird durch thermisches Verdampfen in einer Vakuumkammer eine 20nm dicke Lochtransportschicht des Materials SpMA1 aufgebracht.

Darauf werden die gleichen Schichten durch Vakuumverdampfen aufgebracht wie in Beispiel 1 (15nm Schicht mit 85 Vol% IC1 und 15 Vol% D1, 10nm IC1, 40nm ST1, 3nm LiQ, 100nm Aluminium).

Anschließend werden die OLEDs verkapselt.

Von diesen OLEDs werden 64 Stück hergestellt. Betreibt man diese bei einer Stromdichte von 20mA/cm², so fallen nach 200h Betrieb 17 (also ca. 27%) davon aus. Die OLEDs zeigen CIE x,y Farbkoordinaten von 0.34/0.58, EQE1000=12.8%, U1000=3.3V.

### Vergleichsbeispiel 1

Auf das Substrat wird eine vernetzbare Lochtransportschicht aufgebracht. Sie besteht aus einem Polymer der folgenden Strukturformel, 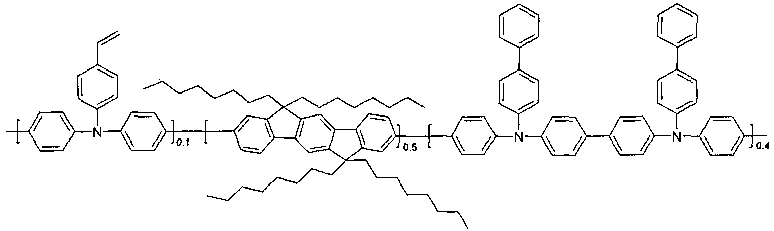 das gemäß WO 2010/097155 synthetisiert wurde. Das Material wird in Toluol gelöst. Der Feststoffgehalt der Lösung liegt bei 5 g/l. Die Schicht wird in einer Inertgasatmosphäre, im vorliegenden Fall Argon, aufgeschleudert und 60 Minuten bei 180°C ausgeheizt. Die Schichtdicke beträgt 20nm.

Darauf werden die gleichen Schichten durch Vakuumverdampfen aufgebracht wie in Beispiel 1 (15nm Schicht mit 85 Vol% IC1 und 15 Vol% D1, 10nm IC1, 40nm ST1, 3nm LiQ, 100nm Aluminium).

Anschließend werden die OLEDs verkapselt.

Von diesen OLEDs werden 64 Stück hergestellt. Betreibt man diese bei einer Stromdichte von 20mA/cm², so fallen nach 200h Betrieb fünf (also ca. 8%) davon aus, also vergleichbar viele wie im Beispiel 1 und deutlich weniger als im Beispiel 2. Die OLEDs zeigen CIE x,y Farbkoordinaten von 0.34/0.59, EQE1000=12.2%, U1000=3.4V. Die Leistungsdaten sind somit wesentlich besser als in Beispiel 1 und vergleichbar mit Beispiel 2.

### Vergleichsbeispiel 2

Auf das Substrat wird eine Lochtransportschicht aufgebracht. Sie besteht aus dem Material SpMA1. Das Material wird in Toluol gelöst. Der Feststoffgehalt der Lösung liegt bei 10 g/l. Die Schicht wird in einer Inertgasatmosphäre, im vorliegenden Fall Argon, aufgeschleudert und 10 Minuten bei 150°C ausgeheizt. Die Schichtdicke beträgt 20nm.

Darauf werden die gleichen Schichten durch Vakuumverdampfen aufgebracht wie im Beispiel 1 (15nm Schicht mit 85 Vol% IC1 und 15 Vol% D1, 10nm IC1, 40nm ST1, 3nm LiQ, 100nm Aluminium).

Anschließend werden die OLEDs verkapselt.

Von diesen OLEDs werden 64 Stück hergestellt. Betreibt man diese bei einer Stromdichte von 20mA/cm², so fallen nach 200h Betrieb neun (also ca. 14%) davon aus, also vergleichbar viele wie im Beispiel 1 und deutlich weniger als im Beispiel 2. Die OLEDs zeigen CIE x,y Farbkoordinaten von 0.34/0.58, EQE1000=13.1%, U1000=3.2V. Die Leistungsdaten sind somit wesentlich besser als in Beispiel 1 und vergleichbar mit Beispiel 2.

### Vergleichsbeispiel 3

Die OLED entspricht dem erfindungsgemäßen Beispiel 1 mit dem Unterschied, dass sich zwischen Substrat und Lochtransportschicht eine 20nm dicke PEDOT:PSS Schicht befindet, die gemäß Beispiel 1 aufgebracht wird.

Von diesen OLEDs werden 64 Stück hergestellt. Betreibt man diese bei einer Stromdichte von 20mA/cm², so fallen nach 200h Betrieb zwei (also ca. 3%) davon aus, also deutlich weniger als in Beispiel 1 und 2. Die OLEDs zeigen CIE x,y Farbkoordinaten von 0.33/0.58, EQE1000=13.6%, U1000=3.3V. Die Leistungsdaten sind somit wesentlich besser als in Beispiel 1 und etwas besser als in Beispiel 2.

### Vergleichsbeispiel 4

Die OLED entspricht dem erfindungsgemäßen Beispiel 2 mit dem Unterschied, dass sich zwischen Substrat und Lochtransportschicht eine 20nm dicke PEDOT:PSS Schicht befindet, die gemäß Beispiel 1 aufgebracht wird.

Von diesen OLEDs werden 64 Stück hergestellt. Betreibt man diese bei einer Stromdichte von 20mA/cm², so fallen nach 200h Betrieb drei (also ca. 5%) davon aus, also deutlich weniger als in Beispiel 1 und 2. Die OLEDs zeigen CIE x,y Farbkoordinaten von 0.34/0.58, EQE1000=14.3%, U1000=3.1V. Die Leistungsdaten sind somit wesentlich besser als in Beispiel 1 und etwas besser als in Beispiel 2.

**Tabelle 1 : Strukturformeln der Materialien für die OLEDs**

| | |
|---|---|
| 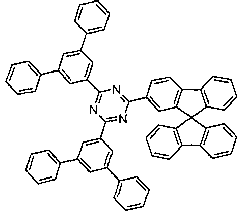 | 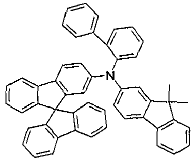 |
| ST1 | SpMA1 |
| 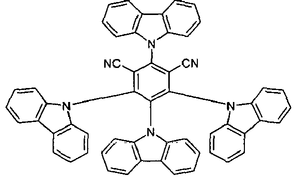 | 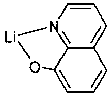 |
| D1 | LiQ |
| 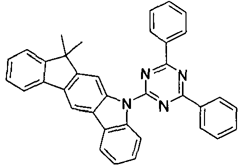 | |
| IC1 | |

### B) Verfahren zur Bestimmung von verwendeten Messwerten (Parametern)

### B-1) Quantenchemisches Verfahren zur Bestimmung von Orbitalenergien und elektronischen Zuständen

Die HOMO- und LUMO- Energien sowie das Triplettniveau und die Singulettniveaus der Materialien werden über quantenchemische Rechnungen bestimmt. Hierzu wird vorliegend das Programmpaket "Gaussian09, Revision D.01" (Gaussian Inc.) verwendet. Zur Berechnung organischer Substanzen ohne Metalle (mit Methode "org." bezeichnet) wird zuerst eine Geometrieoptimierung mit der semi-empirischen Methode AM1 (Gaussian-Eingabezeile "# AM1 opt") mit der Ladung (Charge) 0 und der Multiplizität (Multiplicity) 1 durchgeführt. Im Anschluss erfolgt auf Grundlage der optimierten Geometrie eine Energierechnung (single point) für den elektronischen Grundzustand und das Triplett-Niveau. Hierbei wird die TDDFT-Methode (time dependent density functional theory) B3PW91 mit dem Basissatz 6-31G(d) (Gaussian-Eingabezeile "# B3PW91/6-31G(d) td=(50-50,nstates=4)") verwendet (Ladung 0, Multiplizität 1). Für metallorganische Verbindungen (mit Methode "M-org." bezeichnet) wird die Geometrie mit der Methode Hartree-Fock und dem Basissatz LanL2MB (Gaussian-Eingabezeile "# HF/LanL2MB opt") optimiert (Ladung 0, Multiplizität 1). Die Energierechnung erfolgt, wie oben beschrieben, analog zu der der organischen Substanzen, mit dem Unterschied, dass für das Metallatom der Basissatz "LanL2DZ" und für die Liganden der Basissatz "6-31G(d)" verwendet wird (Gaussian-Eingabezeile "#B3PW91/gen pseudo=lanl2 td=(50-50,nstates=4)"). Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:
HOMO(eV) = ((HEh*27.212)-0.9899)/1.1206
LUMO(eV) = ((LEh*27.212)-2.0041)/1.385

Diese Werte sind im Sinne dieser Anmeldung als HOMO bzw. als LUMO der Materialien anzusehen.

Das Triplett-Niveau T₁ eines Materials ist definiert als die relative Anregungsenergie (in eV) des Triplettzustands mit der niedrigsten Energie, der sich aus der quantenchemischen Energierechnung ergibt.

Das Singulett-Niveau S₁ eines Materials ist definiert als die relative Anregungsenergie (in eV) des Singulettzustands mit der zweitniedrigsten Energie, der sich aus der quantenchemischen Energierechnung ergibt.

Der energetisch niedrigste Singulettzustand wird als S₀ bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian09, Revision D.01" verwendet.

In Tabelle 2 sind die HOMO- und LUMO-Energieniveaus sowie S₁ und T₁ der verschiedenen Materialien angegeben. Tabelle 1 zeigt die Strukturformeln der verwendeten Materialien.

**Tabelle 2: HOMO, LUMO, T₁, S₁ der Materialien**

| Material | Methode | HOMO | LUMO | S₁ | T₁ |
|---|---|---|---|---|---|
| | | (eV) | (eV) | (eV) | (eV) |
| D1 | orp. | -6.11 | -3.40 | 2.50 | 2.41 |
| IC1 | org. | -5.79 | -2.83 | 3.09 | 2.69 |
| SpMA1 | org. | -5.25 | -2.18 | 3.34 | 2.58 |
| ST1 | orp. | -6.03 | -2.82 | 3.32 | 2.68 |
| LiQ | M-org. | -5.17 | -2.39 | 2.85 | 2.13 |

### B-2) Bestimmung der Photolumineszenz-Quanteneffizienz (PLQE)

Von den in den verschiedenen OLEDs verwendeten Emissionsschichten wird ein 50 nm dicker Film auf ein geeignetes transparentes Substrat, vorzugsweise Quarz, aufgebracht, d. h. die Schicht enthält dieselben Materialien in denselben Konzentrationen wie in der OLED. Hierbei werden die gleichen Herstellungsbedingungen wie bei der Herstellung der Emissionsschicht für die OLEDs verwendet. Von diesem Film wird ein Absorptionsspektrum im Wellenlängenbereich von 350-500 nm gemessen. Hierzu wird das Reflexionsspektrum R(λ) sowie das Transmissionsspektrum T(λ) der Probe unter einem Einfallswinkel von 6° (also nahezu senkrechter Einfall) bestimmt. Als Absorptionsspektrum im Sinne dieser Anmeldung wird A(λ)=1-P(λ)-T(λ) definiert.

Gilt A(λ) ≤ 0.3 im Bereich 350-500nm, so wird die zum Maximum des Absorptionsspektrums gehörige Wellenlänge im Bereich 350-500 nm als λ_{exc} definiert. Gilt für irgendeine Wellenlänge A(λ) > 0.3, so wird als Xexc die größte Wellenlänge definiert, bei der A(λ) von einem Wert kleiner 0.3 zu einem Wert größer 0.3 oder von einem Wert größer 0.3 zu einem Wert kleiner 0.3 wechselt.

Zur Bestimmung der PLQE wird ein Messplatz Hamamatsu C9920-02 verwendet. Das Prinzip beruht auf der Anregung der Probe mit Licht definierter Wellenlänge und der Messung der absorbierten und emittierten Strahlung. Die Probe befindet sich während der Messung in einer Ulbrichtkugel ("integrating sphere"). Das Spektrum des Anregungslichts ist in etwa gaußförmig mit einer Halbwertsbreite < 10 nm und Peakwellenlänge λ_{exc} wie oben definiert.

Die PLQE wird nach dem für den genannten Messplatz üblichen Auswerteverfahren bestimmt. Es ist strengstens darauf zu achten, dass die Probe zu keinem Zeitpunkt mit Sauerstoff in Berührung kommt, da die PLQE von Materialien mit kleinem energetischen Abstand zwischen S₁ und T₁ durch Sauerstoff sehr stark reduziert wird (H. Uoyama et al., Nature 2012, Vol. 492, 234).

### B-3) Bestimmung der Abklingzeit

Zur Bestimmung der Abklingzeit wird eine Probe verwendet, die wie oben unter "Bestimmung der PL-Quanteneffizienz (PLQE)" beschrieben hergestellt wird. Die Probe wird bei einer Temperatur von 295 K durch einen Laserpuls angeregt (Wellenlänge 266 nm, Pulsdauer 1.5 ns, Pulsenergie 200 µJ, Strahldurchmesser 4 mm). Die Probe befindet sich hierbei im Vakuum (<10⁻⁵ mbar). Nach der Anregung (definiert als t = 0) wird der zeitliche Verlauf der Intensität der emittierten Photolumineszenz gemessen. Die Photolumineszenz zeigt am Anfang einen steilen Abfall, der auf die prompte Fluoreszenz der TADF-Verbindung zurückzuführen ist. Im weiteren zeitlichen Verlauf ist ein langsamerer Abfall zu beobachten, die verzögerte Fluoreszenz (siehe z.B. H. Uoyama et al., Nature, vol. 492, no. 7428, pp. 234-238, 2012 sowie K. Masui et al., Organic Electronics, vol. 14, no. 11, pp. 2721-2726, 2013). Die Abklingzeit tₐ im Sinne dieser Anmeldung ist die Abklingzeit der verzögerten Fluoreszenz und wird wie folgt bestimmt: Man wählt einen Zeitpunkt t_{d}, zu dem die prompte Fluoreszenz deutlich unter die Intensität der verzögerten Fluoreszenz abgeklungen ist (<1%), so dass die folgende Bestimmung der Abklingzeit davon nicht beeinflusst wird. Diese Wahl kann von einem Fachmann durchgeführt werden. Für die Messdaten ab dem Zeitpunkt t_{d} wird die Abklingzeit ta = te -t_{d} bestimmt. Dabei ist te derjenige Zeitpunkt nach t = t_{d}, bei dem die Intensität erstmals auf 1/e ihres Wertes bei t = t_{d} abgefallen ist.

## Patentansprüche

1. Organische Elektrolumineszenzvorrichtung, enthaltend
- Anode,
- Kathode,
- eine emittierende Schicht, welche eine emittierende Verbindung enthält, die eine betragsmäßige Differenz zwischen den Energien ihres S₁- und T₁-Zustands von nicht mehr als 0.15 eV aufweist, und die sowohl mindestens einen Donorsubstituenten als auch mindestens einen Akzeptorsubstituenten aufweist, wobei das LUMO und das HOMO der Verbindung räumlich nur geringfügig überlappen, wobei der mindestens eine Donorsubstituent gewählt ist aus Diaryl-oder Diheteroarylaminogruppen sowie Carbazolgruppen oder Carbazolderivaten, die jeweils bevorzugt über N an eine aromatische Verbindung gebunden sind, wobei die Gruppen auch weiter substituiert sein können, und wobei der mindestens eine Akzeptorsubstituent gewählt ist aus Cyanogruppen und elektronenarmen Heteroarylgruppen, die auch weiter substituiert sein können, wobei die Energien des S₁- und T₁-Zustands der emittierenden Verbindung bestimmt werden wie in den Ausführungsbeispielen angegeben und
- eine zwischen Anode und emittierender Schicht angeordnete Schicht, welche eine Aminverbindung enthält, welche aus Lösung aufgebracht ist, und welche eine Dicke von mehr als 30 nm hat, wobei die Aminverbindung gewählt ist aus einem Polymer enthaltend Triarylamingruppen, wobei das Polymer mindestens eine Struktureinheit umfasst, die der folgenden Formel (I) entspricht 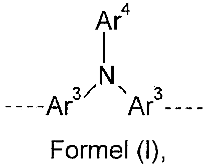 und eine Struktureinheit der folgenden Formel (II), die von der Struktureinheit der Formel (I) verschieden ist:
---- Ar⁵---- Formel (II)
wobei gilt:
Ar³ , Ar⁴ ist bei jedem Auftreten gleich oder verschieden ein aromatisches Ringsystem mit 6 bis 40 aromatischen Ringatomen, das mit einem oder mehreren Resten R³ substituiert sein kann, oder ein heteroaromatisches Ringsystem mit 5 bis 40 aromatischen Ringatomen, das mit einem oder mehreren Resten R³ substituiert sein kann;
R³ ist bei jedem Auftreten gleich oder verschieden gewählt aus H, D, F, Cl, Br, I, B(OR⁴)₂, C(=O)R⁴, CN, Si(R⁴)₃, N(R⁴)2, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch - R⁴C=CR⁴-, -C≡C-, Si(R⁴)₂, C=O , C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, Cl, Br, I, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können;
Ar⁵ ist aus Indenofluorenen gewählt; und
die gestrichelten Linien Bindungen zu benachbarten Struktureinheiten im Polymer darstellen.

2. Organische Elektrolumineszenzvorrichtung nach Anspruch 1, **dadurch gekennzeichnet, dass** die emittierende Verbindung eine lumineszierende Verbindung ist, und dass sie eine Lumineszenzquanteneffizienz, gemessen wie in den Ausführungsbeispielen angegeben, von mindestens 50 % aufweist.

3. Organische Elektrolumineszenzvorrichtung nach Anspruch 1 oder 2, **dadurch gekennzeichnet, dass** die emittierende Verbindung eine betragsmäßige Differenz zwischen den Energien ihres S₁- und T₁-Zustands von nicht mehr als 0.05 eV aufweist.

4. Organische Elektrolumineszenzvorrichtung nach einem oder mehreren der Ansprüche 1 bis 3, **dadurch gekennzeichnet, dass** mindestens eine der Struktureinheiten der Formel (I) mindestens eine vernetzbare Gruppe aufweist.

5. Organische Elektrolumineszenzvorrichtung nach Anspruch 4, **dadurch gekennzeichnet, dass** die vernetzbare Gruppe gewählt ist aus endständigen oder cyclischen Alkenylgruppen, endständigen Dienylgruppen, endständigen Alkinylgruppen, Alkenyloxygruppen, Dienyloxygruppen, Alkinyloxygruppen, Acrylsäuregruppen, Oxetanen, Oxiranen, Silanen, und Cyclobutangruppen.

6. Organische Elektrolumineszenzvorrichtung nach einem oder mehreren der Ansprüche 1 bis 5, **dadurch gekennzeichnet, dass** das Aufbringen der Schicht enthaltend die Aminverbindung aus Lösung durch eine Methode erfolgt, die gewählt ist aus Spincoating und Druckverfahren, beispielsweise Siebdruck, Flexodruck, Nozzle Printing, Offsetdruck, LITI (Light Induced Thermal Imaging, Thermotransferdruck) oder Ink-Jet Druck (Tintenstrahldruck).

7. Organische Elektrolumineszenzvorrichtung nach einem oder mehreren der Ansprüche 1 bis 6, **dadurch gekennzeichnet, dass** die Schicht, welche aus Lösung aufgebracht ist und eine Aminverbindung enthält, direkt auf die Anode aufgebracht ist.

8. Organische Elektrolumineszenzvorrichtung nach einem oder mehreren der Ansprüche 1 bis 7, **dadurch gekennzeichnet, dass** alle Schichten zwischen Anode und emittierender Schicht aus Lösung aufgebracht sind, und dass die emittierende Schicht und alle Schichten zwischen emittierender Schicht und Kathode aus der Gasphase aufgebracht sind.

9. Verwendung einer organischen Elektrolumineszenzvorrichtung nach einem oder mehreren der Ansprüche 1 bis 8 in Displays, als Lichtquelle in Beleuchtungsanwendungen oder als Lichtquelle in medizinischen oder kosmetischen Anwendungen.

10. Verfahren zur Herstellung einer organischen Elektrolumineszenzvorrichtung, enthaltend
- Anode,
- Kathode,
- eine emittierende Schicht, welche eine emittierende Verbindung enthält, die eine Differenz zwischen den Energien ihres S₁- und T₁-Zustands von nicht mehr als 0.15 eV aufweist, wobei die Energien des S₁- und T₁-Zustands der emittierenden Verbindung bestimmt werden wie in den Ausführungsbeispielen angegeben, und die sowohl mindestens einen Donorsubstituenten als auch mindestens einen Akzeptorsubstituenten aufweist, wobei das LUMO und das HOMO der Verbindung räumlich nur geringfügig überlappen, wobei der mindestens eine Donorsubstituent gewählt ist aus Diaryl- oder Diheteroarylaminogruppen sowie Carbazolgruppen oder Carbazolderivaten, die jeweils bevorzugt über N an eine aromatische Verbindung gebunden sind, wobei die Gruppen auch weiter substituiert sein können, und wobei der mindestens eine Akzeptorsubstituent gewählt ist aus Cyanogruppen und elektronenarmen Heteroarylgruppen, die auch weiter substituiert sein können, und
- eine zwischen Anode und emittierender Schicht angeordnete Schicht, welche eine Aminverbindung enthält,
wobei die Aminverbindung gewählt ist aus einem Polymer enthaltend Triarylamingruppen, wobei das Polymer mindestens eine Struktureinheit umfasst, die der folgenden Formel (I) entspricht 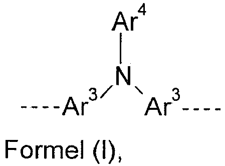 und eine Struktureinheit der folgenden Formel (II), die von der Struktureinheit der Formel (I) verschieden ist:
---- Ar⁵---- Formel (II)
wobei gilt:
Ar³ , Ar⁴ ist bei jedem Auftreten gleich oder verschieden ein aromatisches Ringsystem mit 6 bis 40 aromatischen Ringatomen, das mit einem oder mehreren Resten R³ substituiert sein kann, oder ein heteroaromatisches Ringsystem mit 5 bis 40 aromatischen Ringatomen, das mit einem oder mehreren Resten R³ substituiert sein kann;
R³ ist bei jedem Auftreten gleich oder verschieden gewählt aus H, D, F, Cl, Br, I, B(OR⁴)₂, C(=O)R⁴, CN, Si(R⁴)₃, N(R⁴)2, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, Cl, Br, I, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können; und
Ar⁵ ist aus Indenofluorenen gewählt; und
die gestrichelten Linien Bindungen zu benachbarten Struktureinheiten im Polymer darstellen; und
wobei die zwischen Anode und emittierender Schicht angeordnete Schicht, welche eine Aminverbindung enthält, eine Dicke von mehr als 30 nm hat und aus Lösung aufgebracht wird.

## Claims

1. Organic electroluminescent device comprising
- anode,
- cathode,
- an emitting layer comprising an emitting compound having a magnitude of the difference between the energies of its S₁ and T₁ states of not more than 0.15 eV, and having both at least one donor substituent and at least one acceptor substituent, with only minor spatial overlap between the LUMO and HOMO of the compound, where the at least one donor substituent is chosen from diaryl- or diheteroarylamino groups and carbazole groups or carbazole derivatives, each preferably bonded to an aromatic compound via N, where the groups may also have further substitution, and where the at least one acceptor substituent is chosen from cyano groups and electron-deficient heteroaryl groups which may also have further substitution, and where the energies of the S₁ and T₁ states of the emitting compound are determined as specified in the working examples,
- a layer which is disposed between the anode and emitting layer and comprises an amine compound has a thickness of more than 30 nm and has been applied from solution, where the amine compound is selected from a polymer containing triarylamine groups, where the polymer comprises at least one structural unit corresponding to the following formula (I): 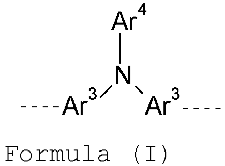 and a structural unit of the following formula (II) which is different from the structural unit of the formula (I):
--- Ar⁵ --- Formula (II)
where:
Ar³, Ar⁴ is the same or different at each instance and is an aromatic ring system which has 6 to 40 aromatic ring atoms and may be substituted by one or more R³ radicals, or a heteroaromatic ring system which has 5 to 40 aromatic ring atoms and may be substituted by one or more R³ radicals;
R³ is the same or different at each instance and is selected from H, D, F, Cl, Br, I, B(OR⁴)₂, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R³ radicals may be joined to one another and may form a ring; where the alkyl, alkoxy, alkenyl and alkynyl groups mentioned and the aromatic ring systems and heteroaromatic ring systems mentioned may each be substituted by one or more R⁴ radicals; and where one or more CH₂ groups in the alkyl, alkoxy, alkenyl and alkynyl groups mentioned may be replaced by -R⁴C=CR⁴-,-C≡C-, Si(R⁴)₂, C=O, C=NR⁴, -C(=O)O-,-C(=O)NR⁴-, NR⁴, P(=O)(R⁴), -O-, -S-, SO or SO₂;
R⁴ is the same or different at each instance and is selected from H, D, F, Cl, Br, I, CN, alkyl groups having 1 to 20 carbon atoms, aromatic ring systems having 6 to 40 aromatic ring atoms and heteroaromatic ring systems having 5 to 40 aromatic ring atoms; where two or more R⁴ radicals may be joined to one another and may form a ring; and where the alkyl groups, aromatic ring systems and heteroaromatic ring systems mentioned may be substituted by F or CN;
Ar⁵ is chosen from indenofluorenes; and
the broken lines represent bonds to adjacent structural units in the polymer.

2. Organic electroluminescent device according to Claim 1, **characterized in that** the emitting compound is a luminescent compound, and **in that** it has a luminescence quantum efficiency, measured as specified in the working examples, of at least 50%.

3. Organic electroluminescent device according to Claim 1 or 2, **characterized in that** the emitting compound has a magnitude of the difference between the energies of its S₁ and T₁ states of not more than 0.05 eV.

4. Organic electroluminescent device according to one or more Claims 1 to 3, **characterized in that** at least one of the structural units of the formula (I) has at least one crosslinkable group.

5. Organic electroluminescent device according to Claim 4, **characterized in that** the crosslinkable group is chosen from terminal or cyclic alkenyl groups, terminal dienyl groups, terminal alkynyl groups, alkenyloxy groups, dienyloxy groups, alkynyloxy groups, acrylic acid groups, oxetanes, oxiranes, silanes and cyclobutane groups.

6. Organic electroluminescent device according to one or more of Claims 1 to 5, **characterized in that** the layer comprising the amine compound is applied from solution by a method chosen from spin-coating and printing methods, for example screen printing, flexographic printing, nozzle printing, offset printing, LITI (light-induced thermal imaging, thermal transfer printing) or inkjet printing.

7. Organic electroluminescent device according to one or more of Claims 1 to 6, **characterized in that** the layer which has been applied from solution and comprises an amine compound has been applied directly to the anode.

8. Organic electroluminescent device according to one or more of Claims 1 to 7, **characterized in that** all the layers between the anode and emitting layer have been applied from solution, and **in that** the emitting layer and all the layers between the emitting layer and the cathode have been applied from the gas phase.

9. Use of an organic electroluminescent device according to one or more of Claims 1 to 8 in displays, as a light source in lighting applications or as a light source in medical or cosmetic applications.

10. Process for producing an organic electroluminescent device comprising
- anode,
- cathode,
- an emitting layer comprising an emitting compound having a difference between the energies of its S₁ and T₁ states of not more than 0.15 eV, where the energies of the S₁ and T₁ states of the emitting compound are determined as specified in the working examples and having both at least one donor substituent and at least one acceptor substituent, with only minor spatial overlap between the LUMO and HOMO of the compound, where the at least one donor substituent is chosen from diaryl- or diheteroarylamino groups and carbazole groups or carbazole derivatives, each preferably bonded to an aromatic compound via N, where the groups may also have further substitution, and where the at least one acceptor substituent is chosen from cyano groups and electron-deficient heteroaryl groups which may also have further substitution,
- a layer which is disposed between the anode and emitting layer and comprises an amine compound, where the amine compound is selected from a polymer containing triarylamine groups, where the polymer comprises at least one structural unit corresponding to the following formula (I): 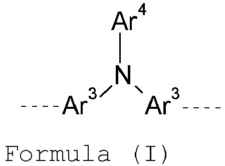 and a structural unit of the following formula (II) which is different from the structural unit of the formula (I):
---- Ar⁵ ---- Formula (II)
where:
Ar³, Ar⁴ is the same or different at each instance and is an aromatic ring system which has 6 to 40 aromatic ring atoms and may be substituted by one or more R³ radicals, or a heteroaromatic ring system which has 5 to 40 aromatic ring atoms and may be substituted by one or more R³ radicals;
R³ is the same or different at each instance and is selected from H, D, F, Cl, Br, I, B(OR⁴)₂, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 carbon atoms, branched or cyclic alkyl or alkoxy groups having 3 to 20 carbon atoms, alkenyl or alkynyl groups having 2 to 20 carbon atoms, aromatic ring systems having 6 to 40 aromatic ring atoms, and heteroaromatic ring systems having 5 to 40 aromatic ring atoms; where two or more R³ radicals may be joined to one another and may form a ring; where the alkyl, alkoxy, alkenyl and alkynyl groups mentioned and the aromatic ring systems and heteroaromatic ring systems mentioned may each be substituted by one or more R⁴ radicals; and where one or more CH₂ groups in the alkyl, alkoxy, alkenyl and alkynyl groups mentioned may be replaced by -R⁴C=CR⁴-,-C≡C-, Si(R⁴)₂, C=O, C=NR⁴, -C(=O)O-,-C(=O)NR⁴-, NR⁴, P(=O)(R⁴), -O-, -S-, SO or SO₂;
R⁴ is the same or different at each instance and is selected from H, D, F, Cl, Br, I, CN, alkyl groups having 1 to 20 carbon atoms, aromatic ring systems having 6 to 40 aromatic ring atoms and heteroaromatic ring systems having 5 to 40 aromatic ring atoms; where two or more R⁴ radicals may be joined to one another and may form a ring; and where the alkyl groups, aromatic ring systems and heteroaromatic ring systems mentioned may be substituted by F or CN;
Ar⁵ is chosen from indenofluorenes; and
the broken lines represent bonds to adjacent structural units in the polymer; and
where the layer which is disposed between the anode and emitting layer and comprises an amine compound has a thickness of more than 30 nm and has been applied from solution.

## Revendications

1. Dispositif organique d'électroluminescence, contenant
- anode,
- cathode,
- une couche émettrice, qui contient un composé émetteur, qui présente une différence en valeur entre les énergies de son état S₁ et T₁ de pas plus de 0,15 eV, et qui présente aussi bien au moins un substituant donneur qu'au moins un substituant accepteur, la LUMO et l'HOMO du composé ne se superposant que légèrement dans l'espace, l'au moins un substituant donneur étant choisi parmi des groupes des diarylamino ou dihétéroarylamino ainsi que des groupes carbazole ou des dérivés de carbazole, qui à chaque fois préférablement sont liés par l'intermédiaire de N à un composé aromatique, les groupes pouvant être également encore substitués, et l'au moins un substituant accepteur étant choisi parmi des groupes cyano et des groupes hétéroaryle pauvres en électrons, qui peuvent également encore être substitués, les énergies de l'état S₁ et T₁ du composé émetteur étant déterminées comme indiqué dans les exemples de réalisation et
- une couche disposée entre l'anode et la couche émettrice, qui contient un composé de type amine, qui est appliqué à partir d'une solution, et qui possède une épaisseur de plus de 30 nm, le composé de type amine étant choisi parmi un polymère contenant des groupes triarylamine, le polymère comprenant au moins un motif structural qui correspond à la formule suivante (I) 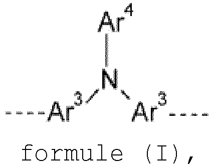 et un motif structural de formule suivante (II), qui est différent du motif structural de formule (I) :
---- Ar⁵ ---- formule (II)
Ar³, Ar⁴, identiques ou différents en chaque occurrence, étant un système cyclique aromatique comportant 6 à 40 atomes de cycle aromatiques, qui peut être substitué par un ou plusieurs radicaux R³, ou un système cyclique hétéroaromatique comportant 5 à 40 atomes de cycle aromatiques, qui peut être substitué par un ou plusieurs radicaux R³ ;
R³, identique ou différent en chaque occurrence, étant choisi parmi H, D, F, Cl, Br, I, B(OR⁴)₂, C(=O)R⁴, CN, Si(R⁴)₃, N(R⁴)₂, P(=O)(R⁴)₂, OR⁴, S(=O)R⁴, S(=O)₂R⁴, des groupes alkyle ou alcoxy linéaires comportant 1 à 20 atome (s) de C, des groupes alkyle ou alcoxy ramifiés ou cycliques comportant 3 à 20 atomes de C, des groupes alcényle ou alcynyle comportant 2 à 20 atomes de C, des systèmes cycliques aromatiques comportant 6 à 40 atomes de cycle aromatiques, et des systèmes cycliques hétéroaromatiques comportant 5 à 40 atomes de cycle aromatiques ; deux radicaux R³ ou plus pouvant être reliés les uns avec les autres et pouvant former un cycle ; et les groupes alkyle, alcoxy, alcényle et alcynyle mentionnés et les systèmes cycliques aromatiques et les systèmes cycliques hétéroaromatiques mentionnés pouvant à chaque fois être substitués par un ou plusieurs radicaux R⁴ ; et un ou plusieurs groupes CH₂ dans les groupes alkyle, alcoxy, alcényle et alcynyle mentionnés pouvant être remplacés par -R⁴C=CR⁴-, - C≡C-, Si(R⁴)₂, C=O, C=NR⁴, -C(=O)O-, -C(=O)NR4-, NR⁴, P(=O)(R⁴), -O-, -S-, SO ou SO₂ ;
R⁴, identique ou différent en chaque occurrence, étant choisi parmi H, D, F, Cl, Br, I, CN, des groupes alkyle comportant 1 à 20 atome (s) de C, des systèmes cycliques aromatiques comportant 6 à 40 atomes de cycle aromatiques et des systèmes cycliques hétéroaromatiques comportant 5 à 40 atomes de cycle aromatiques ; deux radicaux R⁴ ou plus pouvant être reliés les uns avec les autres et pouvant former un cycle ; et les groupes alkyle, les systèmes cycliques aromatiques et les systèmes cycliques hétéroaromatiques mentionnés pouvant être substitués par F ou CN ;
Ar⁵ étant choisi parmi indénofluorène ; et
les lignes en pointillés représentant les liaisons à des motifs structuraux voisins dans le polymère.

2. Dispositif organique d'électroluminescence selon la revendication 1, **caractérisé en ce que** le composé émettreur est un composé luminescent, et **en ce qu'**il présente un rendement quantique de luminescence, mesuré comme indiqué dans les exemples de réalisation, d'au moins 50 %.

3. Dispositif organique d'électroluminescence selon la revendication 1 ou 2, **caractérisé en ce que** le composé émetteur présente une différence en valeur entre les énergies de son état S₁ et T₁ de pas plus de 0,05 eV.

4. Dispositif organique d'électroluminescence selon l'une ou plusieurs des revendications 1 à 3, **caractérisé en ce qu'**au moins un des motifs structuraux de formule (I) présente au moins un groupe réticulable.

5. Dispositif organique d'électroluminescence selon la revendication 4, **caractérisé en ce que** le groupe réticulable est choisi parmi des groupes alcényle terminaux ou cycliques, des groupes diényle terminaux, des groupes alcynyle terminaux, des groupes alcényloxy, des groupes diényloxy, des groupes alcynyloxy, des groupes acide acrylique, des oxétanes, des oxiranes, des silanes et des groupes cyclobutane.

6. Dispositif organique d'électroluminescence selon l'une ou plusieurs des revendications 1 à 5, **caractérisé en ce que** l'application de la couche contenant le composé de type amine à partir d'une solution est réalisée par l'intermédiaire d'une méthode, qui est choisie parmi un revêtement par centrifugation et un procédé d'impression, par exemple de sérigraphie, de flexographie, d'impression par buse, d'impression offset, de LITI (Light Induced Thermal Imaging, imagerie thermique par induction de lumière) ou d'impression ink-jet (d'impression à jet d'encre).

7. Dispositif organique d'électroluminescence selon l'une ou plusieurs des revendications 1 à 6, **caractérisé en ce que** la couche, qui est appliquée à partir d'une solution et contient un composé de type amine, est déposée directement sur l'anode.

8. Dispositif organique d'électroluminescence selon l'une ou plusieurs des revendications 1 à 7, **caractérisé en ce que** toutes les couches entre l'anode et la couche émettrice sont appliquées à partir d'une solution, et **en ce que** la couche émettrice et toutes les couches entre la couche émettrice et la cathode sont appliquées à partir de la phase gazeuse.

9. Utilisation d'un dispositif organique d'électroluminescence selon l'une ou plusieurs des revendications 1 à 8 dans des affichages, en tant que source de lumière dans des applications d'éclairage ou en tant que source de lumière dans des applications médicales ou cosmétiques.

10. Procédé pour la préparation d'un dispositif organique d'électroluminescence, contenant
- anode,
- cathode,
- une couche émettrice, qui contient un composé émetteur, qui présente une différence entre les énergies de son état S₁ et T₁ de pas plus de 0,15 eV, les énergies de l'état S₁ et T₁ du composé émetteur étant déterminées comme indiqué dans les exemples de réalisation, et qui présente aussi bien au moins un substituant donneur qu'au moins un substituant accepteur, la LUMO et l'HOMO du composé ne se superposant que légèrement dans l'espace, l'au moins un substituant donneur étant choisi parmi des groupes des diarylamino ou dihétéroarylamino ainsi que des groupes carbazole ou des dérivés de carbazole, qui à chaque fois préférablement sont liés par l'intermédiaire de N à un composé aromatique, les groupes pouvant être également encore substitués, et l'au moins un substituant accepteur étant choisi parmi des groupes cyano et des groupes hétéroaryle pauvres en électrons, qui peuvent également encore être substitués, et
- une couche disposée entre l'anode et la couche émettrice, qui contient un composé de type amine, le composé de type amine étant choisi parmi un polymère contenant des groupes triarylamine, le polymère comprenant au moins un motif structural qui correspond à la formule suivante (I) 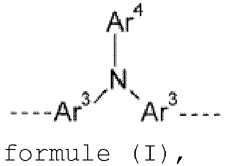 et un motif structural de formule suivante (II), qui est différent du motif structural de formule (I) :
---- Ar⁵ ---- formule (II)
Ar³, Ar⁴, identiques ou différents en chaque occurrence, étant un système cyclique aromatique comportant 6 à 40 atomes de cycle aromatiques, qui peut être substitué par un ou plusieurs radicaux R³, ou un système cyclique hétéroaromatique comportant 5 à 40 atomes de cycle aromatiques, qui peut être substitué par un ou plusieurs radicaux R³ ;
R³, identique ou différent en chaque occurrence, étant choisi parmi H, D, F, Cl, Br, I, B(OR⁴)₂, C(=O)R⁴, CN, Si(R⁴)₃, N(R⁴)₂, P(=O)₂, OR⁴, S(=O)R⁴, S(=O)₂R⁴, des groupes alkyle ou alcoxy linéaires comportant 1 à 20 atome(s) de C, des groupes alkyle ou alcoxy ramifiés ou cycliques comportant 3 à 20 atomes de C, des groupes alcényle ou alcynyle comportant 2 à 20 atomes de C, des systèmes cycliques aromatiques comportant 6 à 40 atomes de cycle aromatiques, et des systèmes cycliques hétéroaromatiques comportant 5 à 40 atomes de cycle aromatiques ; deux radicaux R³ ou plus pouvant être reliés les uns avec les autres et pouvant former un cycle ; et les groupes alkyle, alcoxy, alcényle et alcynyle mentionnés et les systèmes cycliques aromatiques et les systèmes cycliques hétéroaromatiques mentionnés pouvant à chaque fois être substitués par un ou plusieurs radicaux R⁴ ; et un ou plusieurs groupes CH₂ dans les groupes alkyle, alcoxy, alcényle et alcynyle mentionnés pouvant être remplacés par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂ ;
R⁴, identique ou différent en chaque occurrence, étant choisi parmi H, D, F, Cl, Br, I, CN, des groupes alkyle comportant 1 à 20 atome (s) de C, des systèmes cycliques aromatiques comportant 6 à 40 atomes de cycle aromatiques et des systèmes cycliques hétéroaromatiques comportant 5 à 40 atomes de cycle aromatiques ; deux radicaux R⁴ ou plus pouvant être reliés les uns avec les autres et pouvant former un cycle ; et les groupes alkyle, les systèmes cycliques aromatiques et les systèmes cycliques hétéroaromatiques mentionnés pouvant être substitués par F ou CN ;
Ar⁵ étant choisi parmi indénofluorène ; et
les lignes en pointillés représentant les liaisons à des motifs structuraux voisins dans le polymère ; et
la couche disposée entre l'anode et la couche émettrice, qui contient un composé de type amine, possédant une épaisseur de plus de 30 nm et étant appliquée à partir d'une solution.
